# EUROPEAN PATENT SPECIFICATION

(11) **EP 2 418 470 B1**
(45) Date of publication and mention of the grant of the patent: **11.12.2013**
(21) Application number: 10761592.4
(22) Date of filing: 25.03.2010
(51) Int. Cl.: B01D 53/14, C01B 31/20, G01N 30/06

(54) **MEASUREMENT DEVICE, MEASUREMENT METHOD, AND CARBON DIOXIDE RECOVERY SYSTEM**
MESSGERÄT, MESSVERFAHREN UND VERFAHREN ZUR WIEDERGEWINNUNG VON KOHLENDIOXID
DISPOSITIF DE MESURE, PROCÉDÉ DE MESURE, ET SYSTÈME DE RÉCUPÉRATION DE DIOXYDE DE CARBONE

(30) Priority: 08.04.2009 JP 2009093701; 22.07.2009 JP 2009170795; 03.03.2010 JP 2010046923
(43) Date of publication of application: 15.02.2012
(73) Proprietor: Kabushiki Kaisha Toshiba, Minato-ku Tokyo 105-8001 (JP)
(72) Inventor: OGAWA Takashi, Tokyo 105-8001 (JP); HODOTSUKA Masatoshi, Tokyo 105-8001 (JP); OOHASHI Yukio, Tokyo 105-8001 (JP); SAKURAI Manabu, Tokyo 105-8001 (JP); YAMANAKA Susumu, Tokyo 105-8001 (JP); TSUCHIYA Naomi, Tokyo 105-8001 (JP); HIRATA Haruhiko, Tokyo 105-8001 (JP)
(74) Representative: HOFFMANN EITLE
(86) International application number: PCT/JP2010/055218
(87) International publication number: WO 2010/116892

(56) References cited:
- WO-A1-2006/132156
- WO-A1-2008/090931
- JP-A- 61 213 655
- JP-A- 2001 033 437
- JP-A- 2004 323 339
- JP-A- 2010 100 491

## Description

### TECHNICAL FIELD

The present invention relates to a measurement device, a measurement method, and a carbon dioxide recovery system.

### BACKGROUND ART

In recent years, the following method has been studied. This method separates and recovers carbon dioxide from a combustion exhaust gas by allowing the combustion exhaust gas, which is generated by the combustion of fossil fuel, to come into contact with an amine-based absorbent solution and stores the recovered carbon dioxide without discharging the recovered carbon dioxide to the atmosphere, in thermal power plants where a large amount of fossil fuel is used or the like.

Specifically, there is known a carbon dioxide recovery system that includes an absorption tower and a regeneration tower (for example, see Patent Document 1). The absorption tower allows carbon dioxide contained in a combustion exhaust gas to be absorbed in an amine-based absorbent solution. The regeneration tower is supplied with the absorbent solution (rich liquid) having absorbed carbon dioxide from the absorption tower, heats the rich liquid, discharges a carbon dioxide gas from the rich liquid, and regenerates the absorbent solution. A reboiler, which supplies a heat source, is connected to the regeneration tower. An absorbent solution (lean liquid) regenerated in the regeneration tower is supplied to the absorption tower, and the absorbent solution circulates through this system.

It is necessary to make the amount of carbon dioxide which is absorbed in the absorbent solution in the absorption tower correspond to the amount of carbon dioxide which is discharged from the absorbent solution in the regeneration tower in order to stably operate this carbon dioxide recovery system. Accordingly, for example, it is required to adjust the thermal energy input to the reboiler, the discharged amount of deteriorated absorbent solution, the supplied amount of new absorbent solution, and the like while monitoring the carbon dioxide content so that the carbon dioxide content of the lean liquid continues to stably have a desired value at an outlet of the regeneration tower or an inlet of the absorption tower.

However, a titration method, which is generally used as a method of measuring carbon dioxide content, requires a long time (1 to 1.5 hours) for obtaining measurement results. For this reason, it was not possible to obtain the optimal adjustment amount of thermal energy or the like, which is input to the reboiler, from the carbon dioxide content measured by this method, and it was not possible to improve the stability of the operation of the carbon dioxide recovery system.

### CITATION LIST

### PATENT DOCUMENT

Patent Document 1: Japanese Patent Application Laid-Open No. 2004-323339

### DISCLOSURE OF THE INVENTION

### PROBLEM TO BE SOLVED BY THE INVENTION

An object of the invention is to provide a measurement method and a measurement device that can quickly measure the carbon dioxide content of an absorbent solution circulating through a carbon dioxide recovery system, and a carbon dioxide recovery system including the measurement device.

### MEANS FOR SOLVING THE PROBLEM

According to one aspect of the present invention, there is provided a measurement device comprising:
a gasification unit that gasifies an organic solution in which an inorganic gas has been dissolved and discharges the gasified organic solution together with a carrier gas;
an organic gas retention unit that is supplied with a gas discharged from the gasification unit, retains an organic gas and allows an inorganic gas to pass therethrough at a first temperature, and discharges the retained organic gas at a second temperature higher than the first temperature;
an inorganic gas separation unit that separates inorganic components contained in the inorganic gas having passed through the organic gas retention unit, and discharges the inorganic components;
an organic gas separation unit that separates organic components contained in the organic gas discharged from the organic gas retention unit, and discharges the organic components; and
a detection unit that detects the inorganic components discharged from the inorganic gas separation unit and the organic components discharged from the organic gas separation unit.

According to one aspect of the present invention, there is provided a measurement method of measuring components of an organic solution in which an inorganic gas has been dissolved by a measurement device that includes a gasification unit, an organic gas retention unit, a flow passage switching unit, an inorganic gas separation unit, an organic gas separation unit, and a detection unit,
wherein the gasification unit gasifies the organic solution and discharges the gasified organic solution together with a carrier gas,
the organic gas retention unit retains an organic gas contained in the gas discharged from the gasification unit and allows an inorganic gas to pass therethrough at a first temperature, the inorganic gas separation unit separates inorganic components contained in the inorganic gas, which has passed through the organic gas retention unit, and discharges the inorganic components, at a third temperature that is higher than the first temperature and lower than a second temperature where the organic gas retention unit discharges the retained organic gas,
the detection unit detects the inorganic components discharged from the inorganic gas separation unit, the organic gas retention unit discharges the organic gas at the second temperature,
the organic gas separation unit separates organic components contained in the organic gas discharged from the organic gas retention unit, and discharges the organic components, and the detection unit detects the organic components discharged from the organic gas separation unit.

According to one embodiment of the present invention, there is provided a carbon dioxide recovery system comprising:
an absorption tower that allows carbon dioxide contained in
a combustion exhaust gas to be absorbed in an absorbent solution and discharges the absorbent solution containing carbon dioxide;
a regeneration tower that is supplied with the absorbent solution discharged from the absorption tower, removes a carbon dioxide gas containing steam from the absorbent solution, regenerates the absorbent solution, and discharges the absorbent solution;
a regenerative heat exchanger that is provided between the absorption tower and the regeneration tower and heats the absorbent solution, which is discharged from the absorption tower and supplied to the regeneration tower, by using an absorbent solution, which is discharged from the regeneration tower and supplied to the absorption tower, as a heat source; a densimeter that measures the density of an absorbent solution discharged from the absorption tower or an absorbent solution discharged from the regeneration tower;
a gasification unit that gasifies a unit of the absorbent solution and discharges the gasified absorbent solution together with a carrier gas;
an organic gas retention unit that is supplied with a gas discharged from the gasification unit, retains an organic gas and allows an inorganic gas to pass therethrough at a first temperature, and discharges the retained organic gas at a second temperature higher than the first temperature;
an inorganic gas separation unit that separates inorganic components contained in the inorganic gas having passed through the organic gas retention unit, and discharges the inorganic components; an organic gas separation unit that separates organic components contained in the organic gas discharged from the organic gas retention unit, and discharges the organic components;
a detection unit that detects the inorganic components discharged from the inorganic gas separation unit and the organic components discharged from the organic gas separation unit; and a control unit that controls the amount of an absorbent solution which is discharged from the absorption tower and returns to the absorption tower or the amount of an absorbent solution which is discharged from the regeneration tower and returns to the regeneration tower on the basis of the density measured by the densimeter and detection results of the detection unit.

According to one embodiment of the present invention, there is provided a carbon dioxide recovery system comprising:
a gas temperature controller that adjusts the temperature of a combustion exhaust gas and discharges the combustion exhaust gas; an absorption tower that allows carbon dioxide contained in the combustion exhaust gas discharged from the gas temperature controller to be absorbed in an absorbent solution and discharges the absorbent solution containing carbon dioxide;
a regeneration tower that is supplied with the absorbent solution discharged from the absorption tower, removes a carbon dioxide gas containing steam from the absorbent solution, regenerates the absorbent solution, and discharges the absorbent solution;
a regenerative heat exchanger that is provided between the absorption tower and the regeneration tower and heats the absorbent solution, which is discharged from the absorption tower and supplied to the regeneration tower, by using an absorbent solution, which is discharged from the regeneration tower and supplied to the absorption tower, as a heat source;
a densimeter that measures the density of an absorbent solution discharged from the absorption tower;
a gasification unit that gasifies a unit of the absorbent solution and discharges the gasified absorbent solution together with a carrier gas;
an organic gas retention unit that is supplied with a gas discharged from the gasification unit, retains an organic gas and allows an inorganic gas to pass therethrough at a first temperature, and discharges the retained organic gas at a second temperature higher than the first temperature;
an inorganic gas separation unit that separates inorganic components contained in the inorganic gas having passed through the organic gas retention unit, and discharges the inorganic components;
an organic gas separation unit that separates organic components contained in the organic gas discharged from the organic gas retention unit, and discharges the organic components;
a detection unit that detects the inorganic components discharged from the inorganic gas separation unit and the organic components discharged from the organic gas separation unit; and
a control unit that calculates first and second thresholds on the basis of the detection results of the detection unit, performs a control so as to lower the set temperature of the gas temperature controller when the density is lower than the first threshold, and performs a control so as to raise the set temperature of the gas temperature controller when the density is higher than the second threshold.

According to one embodiment of the present invention, there is provided a carbon dioxide recovery system comprising:
an absorption tower that allows carbon dioxide contained in a combustion exhaust gas to be absorbed in an absorbent solution and discharges the absorbent solution containing carbon dioxide;
a regeneration tower that is supplied with the absorbent solution discharged from the absorption tower, removes a carbon dioxide gas containing steam from the absorbent solution, regenerates the absorbent solution, and discharges the absorbent solution;
a reboiler that heats a unit of an absorbent solution stored in the regeneration tower;
a regenerative heat exchanger that is provided between the absorption tower and the regeneration tower and heats the absorbent solution, which is discharged from the absorption tower and supplied to the regeneration tower, by using an absorbent solution, which is discharged from the regeneration tower and supplied to the absorption tower, as a heat source;
a densimeter that measures the density of an absorbent solution discharged from the regeneration tower;
a gasification unit that gasifies a unit of the absorbent solution and discharges the gasified absorbent solution together with a carrier gas;
an organic gas retention unit that is supplied with a gas discharged from the gasification unit, retains an organic gas and allows an inorganic gas to pass therethrough at a first temperature, and discharges the retained organic gas at a second temperature higher than the first temperature;
an inorganic gas separation unit that separates inorganic components contained in the inorganic gas having passed through the organic gas retention unit, and discharges the inorganic components;
an organic gas separation unit that separates organic components contained in the organic gas discharged from the organic gas retention unit, and discharges the organic components;
a detection unit that detects the inorganic components discharged from the inorganic gas separation unit and the organic components discharged from the organic gas separation unit; and
a control unit that calculates first and second thresholds on the basis of the detection results of the detection unit, performs a control so as to lower the set temperature of the reboiler when the density is lower than the first threshold, and performs a control so as to raise the set temperature of the reboiler when the density is higher than the second threshold.

### ADVANTAGE OF THE INVENTION

According to the invention, it is possible to quickly measure the carbon dioxide content of an absorbent solution circulating through a carbon dioxide recovery system.

### BRIEF DESCRIPTION OF THE DRAWINGS

Fig. 1 is a view showing the schematic structure of a measurement device according to a first embodiment of the invention;
Fig. 2 is a view showing the schematic structure of a carbon dioxide recovery system;
Fig. 3 is a flowchart illustrating a method of measuring components of an absorbent solution circulating through the carbon dioxide recovery system by the measurement device according to the first embodiment;
Fig. 4 is a graph showing the results of the component analysis of an absorbent solution circulating through the carbon dioxide recovery system that are measured by the measurement device according to the first embodiment;
Fig. 5 is a view showing the schematic structure of a carbon dioxide recovery system according to a second embodiment of the invention;
Fig. 6 is a view showing the schematic structure of a rich liquid line that branches a part of a flow;
Fig. 7 is a graph showing an example of a relationship between density and the control of the opening of a regulating valve;
Fig. 8 is a view showing the schematic structure of a carbon dioxide recovery system according to a third embodiment of the invention;
Fig. 9 is a view showing the schematic structure of a carbon dioxide recovery system according to a fourth embodiment of the invention; .
Fig. 10 is a view showing the schematic structure of a carbon dioxide recovery system according to a fifth embodiment of the invention;
Fig. 11 is a view showing the schematic structure of a carbon dioxide recovery system according to a sixth embodiment of the invention; and
Fig. 12 is a view showing the schematic structure of a carbon dioxide recovery system according to a seventh embodiment of the invention.

### MODE FOR CARRYING OUT THE INVENTION

Hereinafter, embodiments of the invention will be described with reference to the drawings.

### (First embodiment)

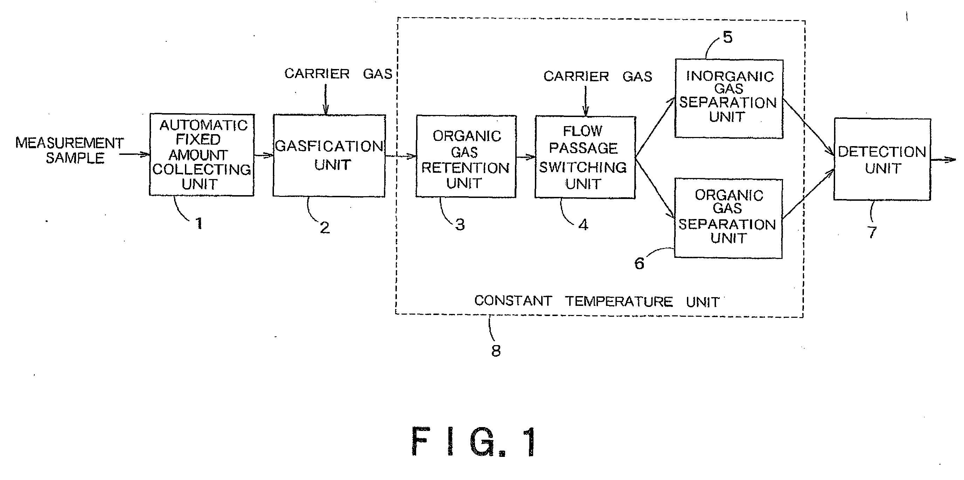

Fig. 1 shows the schematic structure of a measurement device according to a first embodiment of the invention. The measurement device includes an automatic fixed amount collecting unit 1, a gasification unit 2, an organic gas retention unit 3, a flow passage switching unit 4, an inorganic gas separation unit 5, an organic gas separation unit 6, and a detection unit 7. The measurement device analyzes the components of an organic solution in which an inorganic gas (low-molecular gas) has been dissolved. Meanwhile, the organic gas retention unit 3, the flow passage switching unit 4, the inorganic gas separation unit 5, and the organic gas separation unit 6 are received in a constant temperature unit 8, and are maintained at a constant temperature. The temperature of the constant temperature unit 8 can be adjusted.

The automatic fixed amount collecting unit 1 automatically collects a fixed amount of a measurement sample from an organic solution that is a component analysis object.

The gasification unit 2 gasifies the measurement sample, which is collected by the automatic fixed amount collecting unit 1, and discharges the measurement sample together with a carrier gas. For example, helium is used as the carrier gas.

The organic gas retention unit 3 temporarily retains an organic gas contained in an exhaust gas that is discharged from the gasification unit 2, and allows an inorganic gas to pass therethrough. Accordingly, an organic gas and an inorganic gas, which are contained in the exhaust gas discharged from the gasification unit 2, are separated from each other by the organic gas retention unit 3.

The organic gas retention unit 3 retains an organic gas at a low temperature, and discharges the retained organic gas at a high temperature. Accordingly, the organic gas retention unit 3 discharges an inorganic gas contained in the exhaust gas discharged from the gasification unit 2 when the temperature of the constant temperature unit 8 is made low, and discharges an organic gas when the temperature of the constant temperature unit 8 is made high. A trap pipe, which includes a filler capable of adsorbing organic components, may be used in the organic gas retention unit 3.

The flow passage switching unit 4 switches a flow passage so that the gas discharged from the organic gas retention unit 3 is supplied to the inorganic gas separation unit 5 or the organic gas separation unit 6. Further, the flow passage switching unit supplies a carrier gas to the inorganic gas separation unit 5 or the organic gas separation unit 6 to which the gas discharged from the organic gas retention unit 3 is not supplied. The carrier gas is, for example, a helium gas.

When an inorganic gas is discharged from the organic gas retention unit 3, that is, when the temperature of the constant temperature unit 8 is low, the flow passage switching unit 4 supplies the inorganic gas, which is discharged from the organic gas retention unit 3, to the inorganic gas separation unit 5 and supplies a carrier gas to the organic gas separation unit 6.

Further, when an organic gas is discharged from the organic gas retention unit 3, that is, when the temperature of the constant temperature unit 8 is high, the flow passage switching unit 4 supplies the organic gas, which is discharged from the organic gas retention unit 3, to the organic gas separation unit 6 and supplies a carrier gas to the inorganic gas separation unit 5.

Since a carrier gas flows in the separation part not in use as described above, it is possible to prevent the inorganic gas separation unit 5 from being contaminated by an organic gas and to prevent the organic gas separation unit 6 from being contaminated by an inorganic gas.

The inorganic gas separation unit 5 is supplied with an inorganic gas, which has passed through the organic gas retention unit 3, through the flow passage switching unit 4. The inorganic gas separation part separates inorganic components in the inorganic gas, and supplies the inorganic components to the detection unit 7. The retention times where the inorganic gas separation unit 5 retains a plurality of inorganic components are different from each other, and the inorganic gas separation part separates the respective inorganic components by supplying the respective inorganic components to the detection unit 7 at different times. A trap pipe, which includes a filler capable of adsorbing inorganic components, may be used in the inorganic gas separation unit 5.

The organic gas separation unit 6 is supplied with an organic gas, which has been discharged from the organic gas retention unit 3, through the flow passage switching unit 4. The organic gas separation part separates organic components in the organic gas, and supplies the organic components to the detection unit 7. The retention times where the organic gas separation unit 6 retains a plurality of organic components are different from each other, and the organic gas separation part separates the respective organic components by supplying the respective organic components to the detection unit 7 at different times. A trap pipe, which includes a filler capable of adsorbing organic components (for example, an amine component), may be used in the organic gas separation unit 6.

The detection unit 7 detects the inorganic components supplied from the inorganic gas separation unit 5 and the organic components supplied from the organic gas separation unit 6. For example, a thermal conductivity detector (TDC) may be used in the detection unit 7. The results of the detection of the components contained in the measurement sample, which is performed by the detection unit 7, are displayed on a display part (not shown). An operator can grasp the components of the organic solution, which is an analysis object, from the displayed results of the detection.

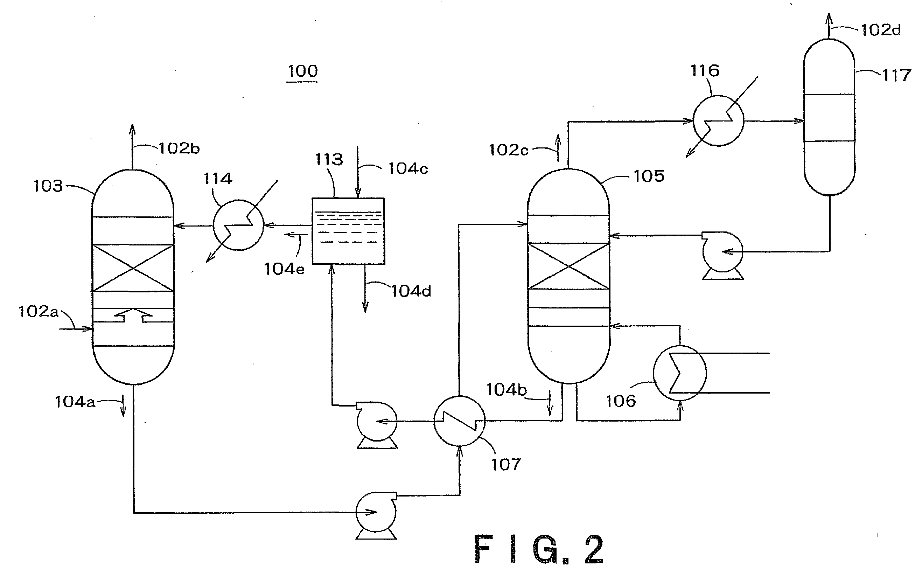

It is possible to analyze the components of an absorbent solution, which circulates through, for example, a carbon dioxide recovery system 100 shown in Fig. 2, by the measurement device. The carbon dioxide recovery system 100 includes an absorption tower 103 and a regeneration tower 105. The absorption tower 103 allows carbon dioxide, which is contained in a combustion exhaust gas 102a, to be absorbed in an absorbent solution. The regeneration tower 105 is supplied with the absorbent solution, which has absorbed carbon dioxide, (hereinafter, referred to as a rich liquid 104a) from the absorption tower 103; discharges a carbon dioxide gas, which contains steam, from the absorbent solution by heating the rich liquid 104a; discharges an exhaust gas 102c that contains a carbon dioxide gas and steam; and regenerates an absorbent solution.

For example, the combustion exhaust gas 102a, which is generated in a power-generating facility such as a thermal power plant, is supplied to the lower portion of the absorption tower 103, and a combustion exhaust gas 102b from which carbon dioxide has been removed is discharged from the top portion of the absorption tower 103. For example, an amine compound aqueous solution, which is obtained by dissolving an amine compound in water, is used as the absorbent solution that can absorb carbon dioxide.

A reboiler 106 generates steam by heating a part of a lean liquid 104b, which is stored in a regeneration tower tank 105, so as to allow the temperature of the lean liquid to rise, and supplies the steam to the regeneration tower 105. Meanwhile, when the lean liquid 104b is heated in the reboiler 106, a small amount of a carbon dioxide gas is discharged from the lean liquid 104b and supplied to the regeneration tower 105 together with the steam. Further, the rich liquid 104a is heated in the regeneration tower 105 by this steam, so that a carbon dioxide gas is discharged.

A regenerative heat exchanger 107, which heats the rich liquid 104a supplied to the regeneration tower 105 from the absorption tower 103 by using the lean liquid 104b supplied to the absorption tower 103 from the regeneration tower 105 as a heat source, is provided between the absorption tower 103 and the regeneration tower 105. Accordingly, the heat of the lean liquid 104b is recovered.

The lean liquid 104b from the regenerative heat exchanger 107 is fed to a tank 113. The tank 113 stores the absorbent solution circulating through the carbon dioxide recovery system 100, is supplied with a new absorbent solution 104c from the upper portion thereof, and discards the absorbent solution 104d from the bottom portion thereof. Accordingly, it is possible to prevent a deteriorated absorbent solution from circulating through the carbon dioxide recovery system 100.

An absorbent solution cooler 114, which cools a lean liquid 104e to be supplied from the tank 113, is provided between the tank 113 and the absorption tower 103. The lean liquid 104e, which has been cooled by the absorbent solution cooler 114, is supplied to the upper portion of the absorption tower 103.

The lean liquid 104e, which is supplied to the upper portion of the absorption tower 103, descends from the upper portion in the absorption tower 103. Meanwhile, the combustion exhaust gas 102a, which is supplied to the absorption tower 103, ascends from the lower portion toward the top portion in the absorption tower 103. For this reason, the lean liquid 104e and the combustion exhaust gas 102a containing carbon dioxide come into countercurrent contact (direct contact) with each other, so that carbon dioxide is removed from the combustion exhaust gas 102a and absorbed in the lean liquid 104e. As a result, the rich liquid 104a is generated. The combustion exhaust gas 102b from which carbon dioxide has been removed is discharged from the top portion of the absorption tower 103.

A condenser 117 separates a generated condensate from a carbon dioxide gas by condensing (cooling) the exhaust gas 102c that contains steam and a carbon dioxide gas discharged from the regeneration tower 105. A carbon dioxide gas 102d, which is discharged from the condenser 117, is stored in a storage facility (not shown).

A gas cooler 116 cools the exhaust gas 102c, which is discharged from the regeneration tower 105, by cooling water (cooling medium). Further, the condensate from the condenser 117 is supplied to the upper portion of the regeneration tower 105.

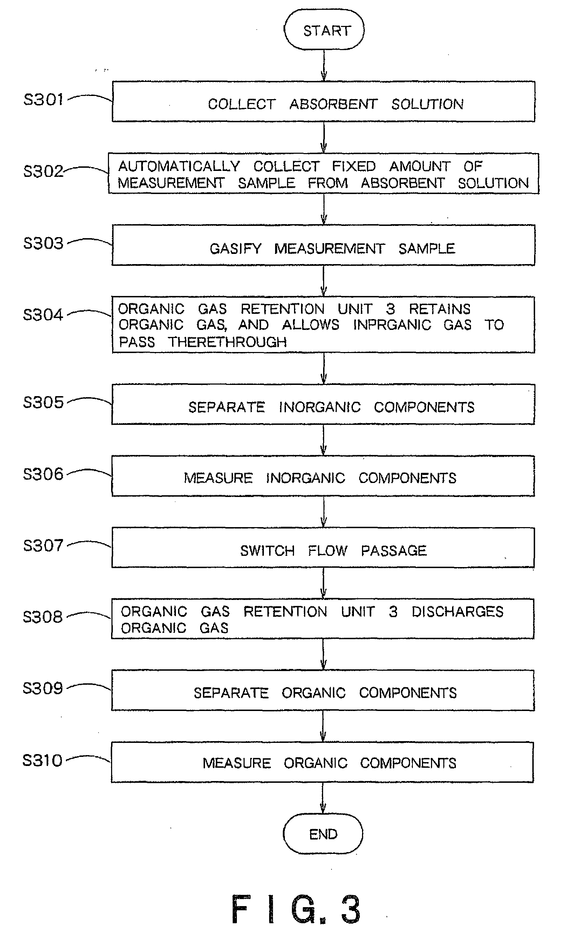

A method of analyzing components of an absorbent solution circulating through the carbon dioxide recovery system 100, which is shown in Fig. 2, by the measurement device according to this embodiment will be described with reference to a flowchart shown in Fig. 3.

### (Step S301)

An absorbent solution circulating through the carbon dioxide recovery system 100 is collected. For example, the absorbent solution (rich liquid 104a), which is supplied to the regeneration tower 105 from the absorption tower 103, is collected.

### (Step S302)

The automatic fixed amount collecting unit 1 automatically collects a fixed amount of a measurement sample from the absorbent solution that is collected in Step S301.

### (Step S303)

The gasification unit 2 gasifies the measurement sample at 270°C, and supplies the measurement sample to the organic gas retention unit 3 together with a carrier gas (helium). A temperature where a liquid sample is to be gasified is set to be equal to or higher than +10°C the highest boiling point of a component to be analyzed.

Further, at this time, the temperature of the constant temperature unit 8 is maintained at 70°C.

### (Step 5304)

The organic gas retention unit 3 retains an organic gas contained in the gas discharged from the gasification unit 2, and allows an inorganic gas to pass therethrough. The inorganic gas is supplied to the inorganic gas separation unit 5 through the flow passage switching unit 4. At this time, the carrier gas (helium) is supplied to the organic gas separation unit 6.

### (Step S305)

The temperature of the constant temperature unit 8 is raised from 70°C to 190°C. Accordingly, inorganic components (carbon dioxide and water vapor) are separated in the inorganic gas separation unit 5. Meanwhile, the temperature of the constant temperature unit 8 is set to be equal to or higher than a temperature where water vapor is completely discharged and to be lower than a temperature where organic components are separated from the organic gas retention unit 3.

### (Step S306)

The inorganic components, which have been separated in Step S305, are measured in the detection unit 7. The analysis temperature of the detection unit 7 (thermal conductivity detector) was set to 270°C.

### (Step S307)

The flow passage of the flow passage switching unit 4 is switched so that a gas from the organic gas retention unit 3 is supplied to the organic gas separation unit 6 and a carrier gas is supplied to the inorganic gas separation unit 5.

### (Step S308)

The temperature of the constant temperature unit 8 is raised from 190°C to 240°C. Accordingly, the organic components retained in the organic gas retention unit 3 are discharged and supplied to the organic gas retention unit 3. Meanwhile, the temperature of the constant temperature unit 8 is set to be equal to or higher than the highest boiling point of a component to be analyzed.

### (Step S309)

Organic components (amines) are separated in the organic gas separation unit 6.

### (Step S310)

The organic components, which have been separated in Step S309, are measured in the detection unit 7.

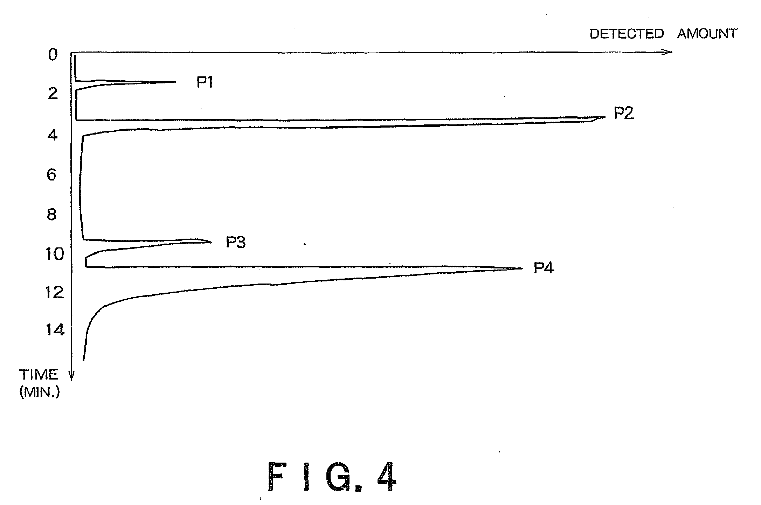

Measurement results shown in Fig. 4 were obtained by this method. In Fig. 4, a peak P1 represents carbon dioxide, a peak P2 represents water, and peaks P3 and P4 represent amines. The measurement results are obtained within 15 minutes, and it is understood that 15 minutes is very shorter than the required time of a titration method (required time: 1 to 1.5 hours).

As described above, it is possible to quickly measure the carbon dioxide content of an absorbent solution circulating through the carbon dioxide recovery system by the measurement device according to this embodiment. Further, it is possible to quickly measure not only the carbon dioxide content of the absorbent solution but also the water content or the organic component (amines) content.

An example where the components of an absorbent solution (rich liquid 104a) are analyzed at the outlet of the absorption tower 103 has been described in the above-mentioned embodiment. However, it is possible to analyze the components of an absorbent solution at various positions in the carbon dioxide recovery system 100. For example, the components of an absorbent solution may be analyzed at the inlet of the absorption tower 103 or the outlet of the regeneration tower 105.

Further, thermal energy input to the reboiler 106, the amount of a new absorbent solution 104c supplied to the tank 113, the amount of the absorbent solution 104d discarded from the tank 113, and the like may be controlled on the basis of the results of the component analysis at a plurality of positions.

For example, when the difference between the carbon dioxide content of an absorbent solution at the outlet of the absorption tower 103 and the carbon dioxide content of an absorbent solution at the outlet of the regeneration tower 105 is larger than the difference between the carbon dioxide content of an absorbent solution at the outlet of the absorption tower 103 and the carbon dioxide content of an absorbent solution at the inlet of the absorption tower 103, thermal energy, which is more than necessary, is input to the reboiler 106. For this reason, the thermal energy, which is to be input to the reboiler 106, is controlled so as to be small. Since it is possible to quickly analyze the components of an absorbent solution by the measurement device according to this embodiment, it is possible to set the thermal energy, which is to be input to the reboiler 106, to an optimal value and to reduce operating cost.

Moreover, whether abnormalities occur or not may be monitored by the component analysis of an absorbent solution at a plurality of positions (an upper portion, a middle portion, and a lower portion) of the absorption tower 103 or the regeneration tower 105. Since it is possible to quickly analyze the components of an absorbent solution by the measurement device according to this embodiment, it is possible to quickly find abnormalities and to improve the stability of the operation of the carbon dioxide recovery system 100.

### (Second embodiment)

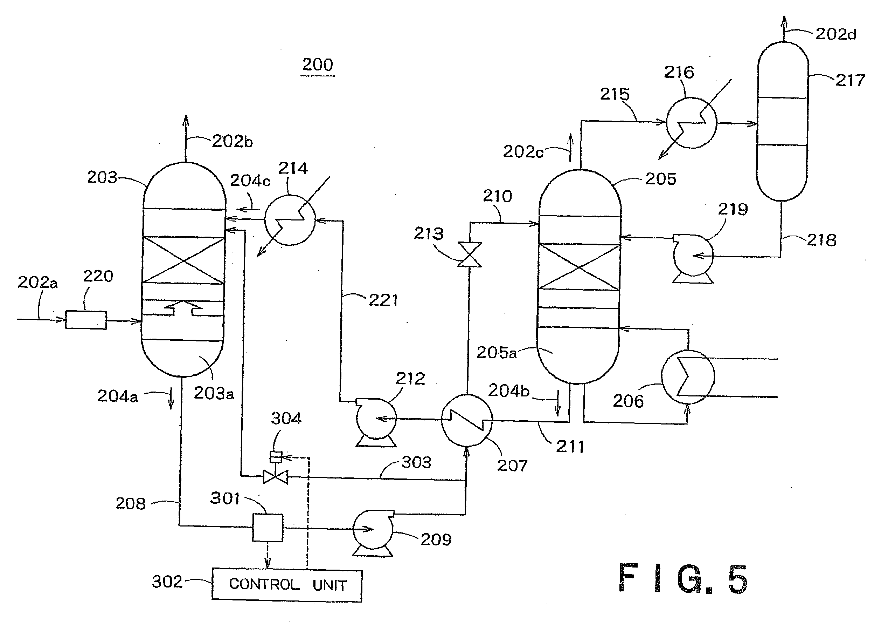

Fig. 5 shows the schematic structure of a carbon dioxide recovery system according to a second embodiment of the invention. Here, the carbon dioxide recovery system recovers carbon dioxide, which is contained in a combustion exhaust gas generated by the combustion of fossil fuel, by using an absorbent solution that can absorb carbon dioxide.

As shown in Fig. 5, the carbon dioxide recovery system 200 includes an absorption tower 203 and a regeneration tower 205. The absorption tower 203 allows carbon dioxide, which is contained in a combustion exhaust gas 202a, to be absorbed in an absorbent solution. The regeneration tower 205 is supplied with the absorbent solution, which has absorbed carbon dioxide, (hereinafter, referred to as a rich liquid 204a) from the absorption tower 203; discharges a carbon dioxide gas, which contains steam, from the absorbent solution by heating the rich liquid 204a; discharges an exhaust gas 202c that contains a carbon dioxide gas and steam; and regenerates an absorbent solution. For example, the combustion exhaust gas 202a, which is generated in a power-generating facility such as a thermal power plant, is supplied to the lower portion of the absorption tower 203, and a combustion exhaust gas 202b from which carbon dioxide has been removed is discharged from the top portion of the absorption tower 203.

The absorption tower 203 includes an absorption tower tank 203a for storing the rich liquid 204a that is generated by allowing the absorbent solution to absorb carbon dioxide. Likewise, the regeneration tower 205 includes a regeneration tower tank 205a for storing the absorbent solution that is regenerated by allowing the rich liquid 204a to discharge a carbon dioxide gas (hereinafter, referred to as a lean liquid 204b). The rich liquid 204a is an absorbent solution having a high carbon dioxide content, and the lean liquid 204b is an absorbent solution having a low carbon dioxide content.

Here, for example, an amine compound aqueous solution, which is obtained by dissolving an amine compound in water, is used as the absorbent solution that can absorb carbon dioxide. The concentration of the amine compound aqueous solution is set to a value that is suitable for the separation and recovery of carbon dioxide.

As shown in Fig. 5, the regeneration tower 205 is provided with a reboiler 206. The reboiler 206 allows the temperature of the lean liquid 204b to rise and generates steam by heating a part of the lean liquid 204b, which is stored in the regeneration tower tank 205a, by using plant steam, which is supplied from a power-generating facility, or the like as a heat source. Then, the reboiler 206 supplies the steam to the regeneration tower 205. Meanwhile, when the lean liquid 204b is heated in the reboiler 206, a carbon dioxide gas is discharged from the lean liquid 204b and supplied to the regeneration tower 205 together with steam. Further, the rich liquid 204a is heated in the regeneration tower 205 by this steam, so that a carbon dioxide gas is discharged.

A condenser 217, which separates a generated condensate (condensed water) from a carbon dioxide gas by condensing (cooling) the exhaust gas 202c containing steam and a carbon dioxide gas discharged from the regeneration tower 205, is connected to the regeneration tower 205. A carbon dioxide gas 202d, which is discharged from the condenser 217, is stored in a storage facility (not shown).

A gas cooling line 215 through which the exhaust gas 202c discharged from the regeneration tower 205 is supplied to the condenser 217 is connected between the regeneration tower 205 and the condenser 217, and a gas cooler 216, which cools the exhaust gas 202c by using cooling water (cooling medium), is provided on the gas cooling line 215. Further, a condensate line 218 through which a condensate from the condenser 217 is supplied to the upper portion of the regeneration tower 205 is connected between the condenser 217 and the regeneration tower 205. A condensate pump 219, which feeds a condensate from the condenser 217 to the regeneration tower 205, is provided on the condensate line 218.

A regenerative heat exchanger 207 is provided between the absorption tower 203 and the regeneration tower 205, and the regenerative heat exchanger 207 heats the rich liquid 204a, which is supplied to the regeneration tower 205 from the absorption tower 203, by using the lean liquid 204b, which is supplied to the absorption tower 203 from the regeneration tower 205, as a heat source. Accordingly, the heat of the lean liquid 204b is recovered. Here, when a carbon dioxide gas is discharged from the rich liquid 204a in the regeneration tower 205, the rich liquid 204a is heated by using high-temperature steam, which is supplied from the reboiler 206, as a heat source as described above. Accordingly, the temperature of the lean liquid 204b, which is supplied to the regenerative heat exchanger 207, is relatively high, and the lean liquid 204b is used as a heat source.

A first rich liquid line 208 through which the rich liquid 204a is supplied to the regenerative heat exchanger 207 from the bottom portion of the absorption tower tank 203a is connected between the absorption tower 203 and the regenerative heat exchanger 207. A rich liquid pump 209, which feeds the rich liquid 204a from the absorption tower 203 to the regenerative heat exchanger 207, is provided on the first rich liquid line 208.

Further, a densimeter 301, which measures the density of the rich liquid 204a in real time, is provided on the first rich liquid line 208. As long as being capable of measuring the density of a liquid fluid in real time, any type of densimeter may be used as the densimeter 301.

For example, a Coriolis mass flowmeter may be used as the densimeter 301. In this case, a portion of the first rich liquid line 208 on which the densimeter 301 (Coriolis mass flowmeter) is mounted may be formed in U shape. The Coriolis mass flowmeter vibrates a pipe while allowing the rich liquid 204a to flow through the pipe (first rich liquid line 208). Since the direction of the flow of a fluid (rich liquid 204a) at the inlet side of the pipe is opposite to the direction of the flow of the fluid at the outlet side of the pipe, Coriolis forces in opposite directions are generated and torsion is generated at the pipe. The amount of torsion is proportional to a mass flow rate. Further, since the frequency of the pipe depends on the density of the fluid, the density of the fluid (rich liquid 204a) is calculated from the frequency of the pipe. Since quickly obtaining the frequency of the pipe (first rich liquid line 208), the Coriolis mass flowmeter can measure the density of the rich liquid 204a substantially in real time.

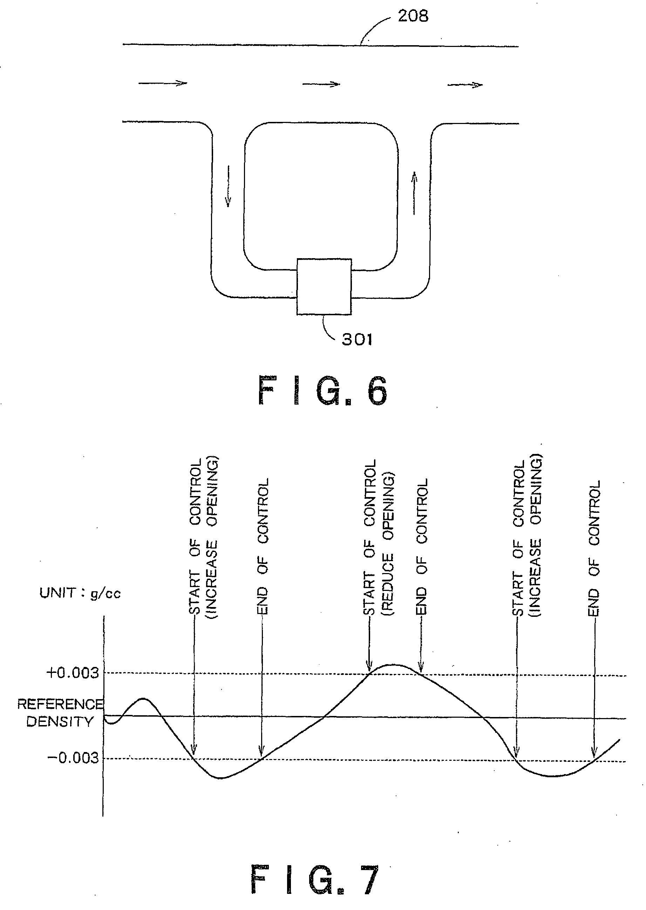

When the speed of a flow is high, a pressure loss of the Coriolis flowmeter is increased. Accordingly, when the density of the rich liquid 204a is to be measured, the total amount of the flow of the rich liquid 204a does not pass through the Coriolis flowmeter and a part of the flow may be branched as shown in Fig. 6 so that a small amount of the rich liquid 204a passes through the Coriolis flowmeter.

The densimeter 301 notifies a control unit 302 of the measured density of the rich liquid 204a.

A rich liquid return line 303 through which the rich liquid 204a returns to the upper portion of the absorption tower 203 (an upper portion above the filler in the absorption tower 203) is connected to the first rich liquid line 208. Here, the diameter of a pipe of the rich liquid return line 303 is set to about 1/2 to 1/5 of the diameter of a pipe of the first rich liquid line 208. The rich liquid 204a, which returns to the absorption tower 203 by the rich liquid return line 303, absorbs carbon dioxide from the combustion exhaust gas 202a again.

A regulating valve 304 is provided on the rich liquid return line 303, and the flow rate of the rich liquid 204a returning to the absorption tower 203 can be adjusted by the opening of the regulating valve 304. The control unit 302 controls the opening of the regulating valve 304 on the basis of the density of the rich liquid 204a. The control unit 302 may calculate the carbon dioxide content of the rich liquid 204a from the density, and may control the opening of the regulating valve 304 on the basis of the result of the calculation. For example, a relationship between the density of the absorbent solution, which is in use, and the carbon dioxide content may be previously obtained and stored in a storage unit (not shown), and the control unit 302 may calculate the carbon dioxide content of the rich liquid 204a with reference to the information stored in the storage unit.

A method of controlling the opening of the regulating valve 304 will be described later.

A second rich liquid line 210, which supplies the rich liquid 204a to the upper portion of the regeneration tower 205 from the regenerative heat exchanger 207, is connected between the regenerative heat exchanger 207 and the regeneration tower 205. A valve 213, which retains the high pressure of the regeneration tower and prevents the absorbent solution from reversely flowing from the regeneration tower at the time of the stop of the pump 209 or the like, is provided on the second rich liquid line 210. When the pressure of the rich liquid is increased by the pump 209, carbon dioxide is separated from the rich liquid in the regenerative heat exchanger 207. Accordingly, the rich liquid is changed into a two-phase flow, so that the reduction of heat exchange efficiency is suppressed.

A first lean liquid line 211, which supplies the lean liquid 204b to the regenerative heat exchanger 207 from the bottom portion of the regeneration tower tank 205a, is connected between the regeneration tower 205 and the regenerative heat exchanger 207.

The lean liquid 204b from the regenerative heat exchanger 207 is fed to an absorbent solution cooler 214 by a lean liquid pump 212 that is provided on a second lean liquid line 221. The absorbent solution cooler 214 cools the lean liquid 204b by using cooling water (cooling medium) as a cooling source. A lean liquid 204c, which has been cooled by the absorbent solution cooler 214, is supplied to the upper portion of the absorption tower 203.

The lean liquid 204c, which is supplied to the upper portion of the absorption tower 203, descends from the upper portion toward the absorption tower tank 203a in the absorption tower 203. After the temperature of the combustion exhaust gas 202a containing about 5 to 20% of carbon dioxide is controlled to a predetermined temperature by a gas temperature controller 220, the combustion exhaust gas 202a is supplied to the lower portion of the absorption tower 203 and ascends from the lower portion toward the top portion in the absorption tower 203. For this reason, the lean liquid and the combustion exhaust gas 202a containing carbon dioxide come into countercurrent contact (direct contact) with each other, so that carbon dioxide is removed from the combustion exhaust gas 202a and absorbed in the lean liquid. As a result, the rich liquid 204a is generated. The combustion exhaust gas 202b from which carbon dioxide has been removed is discharged from the top portion of the absorption tower 203, and the rich liquid 204a is stored in the absorption tower tank 203a of the absorption tower 203.

The carbon dioxide recovery system requires reducing the amount of heat input to the reboiler 206 of the regeneration tower 205 while recovering 50% or more, preferably, 90% or more of carbon dioxide contained in the combustion exhaust gas 202a in the absorption tower 203. For this purpose, it is necessary to control the flow rate, temperature, composition, and pressure of the absorbent solution to optimal values at each portion of the carbon dioxide recovery system.

The lean liquid 204c having a low carbon dioxide content and the combustion exhaust gas 202a come into gas-liquid contact with each other in the absorption tower 203 and the carbon dioxide content of the absorbent solution is increased, so that the lean liquid is changed into the rich liquid 204a. Carbon dioxide is separated from the rich liquid 204a, which is transferred to the regeneration tower 205 from the absorption tower 203, by heating and the carbon dioxide content of the rich liquid is reduced, so that the rich liquid is changed into the lean liquid 204b. The lean liquid 204b is supplied again to the absorption tower 203. Accordingly, the carbon dioxide content of the rich liquid 204a and/or the lean liquid 204b is an important parameter in the optimal operation of the carbon dioxide recovery system.

This embodiment is focused on the carbon dioxide content of the rich liquid 204a (the density related to the carbon dioxide content), and is to improve operation stability and reduce the amount of heat supplied to the reboiler 206 while securing a target recovery rate of carbon dioxide by controlling the carbon dioxide content.

A method of controlling the opening of the regulating valve 304 by the control unit 302 according to this embodiment will be described.

If the density of the rich liquid 204a notified by the densimeter 301 is lower than a predetermined value, that is, if the carbon dioxide content of the rich liquid 204a is low, a desired amount of carbon dioxide is not absorbed in the absorbent solution in the absorption tower 203. For this reason, a target recovery rate of carbon dioxide is not secured. Meanwhile, a fact that much time does not pass after the replacement of an absorbent solution, a fact that an absorbent solution is deteriorated, or the like is considered as the cause of the reduction of the density of the rich liquid 204a.

In this case, the control unit 302 increases the opening of the regulating valve 304 to increase the flow rate of the rich liquid return line 303.

Since the rich liquid 204a having returned to the absorption tower 203 absorbs carbon dioxide from the combustion exhaust gas 202a again, a desired amount of carbon dioxide can be absorbed in the absorbent solution and the density of the rich liquid is increased. Accordingly, it is possible to secure a target recovery rate of carbon dioxide. Meanwhile, the flow rate of the pump 209 may be increased so that the flow rate of the rich liquid 204a supplied to the regeneration tower 205 is not reduced.

On the other hand, if the density of the rich liquid 204a notified by the densimeter 301 is higher than a predetermined value, that is, if the carbon dioxide content of the rich liquid 204a is high, carbon dioxide more than a desired amount is absorbed in the absorbent solution. For this reason, carbon dioxide cannot be sufficiently separated from the absorbent solution in the regeneration tower 205. In this case, the control unit 302 reduces the opening of the regulating valve 304 to reduce the flow rate of the rich liquid return line 303.

Since the amount of the absorbent solution, which has a high carbon dioxide content and circulates through the absorption tower 203, is reduced, the carbon dioxide content of the rich liquid 204a discharged from the absorption tower 203 is reduced and the density of the rich liquid is reduced. Accordingly, it is possible to sufficiently separate carbon dioxide from the absorbent solution in the regeneration tower 205 without increasing the amount of heat supplied to the reboiler 206. Meanwhile, the flow rate of the pump 209 may be reduced so that the flow rate of the rich liquid 204a supplied to the regeneration tower 205 is not increased.

Fig. 7 shows an example of the control timing of the opening of the regulating valve 304 and the temporal change of the density of the rich liquid 204a. When the density of the rich liquid 204a is reduced from predetermined reference density by 0.003 g/cc or more, the opening of the regulating valve 304 is increased. When the density of the rich liquid 204a is increased from predetermined reference density by 0.003 g/cc or more, the opening of the regulating valve 304 is reduced.

In this embodiment, the flow rate of the rich liquid 204a returning to the absorption tower 203 is adjusted according to the density of the rich liquid 204a as described above. Accordingly, the amount of heat supplied to the reboiler 206 is reduced while a target recovery rate of carbon dioxide is secured. Since the density of the rich liquid 204a is obtained in real time by the densimeter 301 as described above, it is possible to quickly reflect density measurement results on the control of the flow rate of the rich liquid 204a returning to the absorption tower 203 and to improve the operation stability of the carbon dioxide recovery system.

### (Third embodiment)

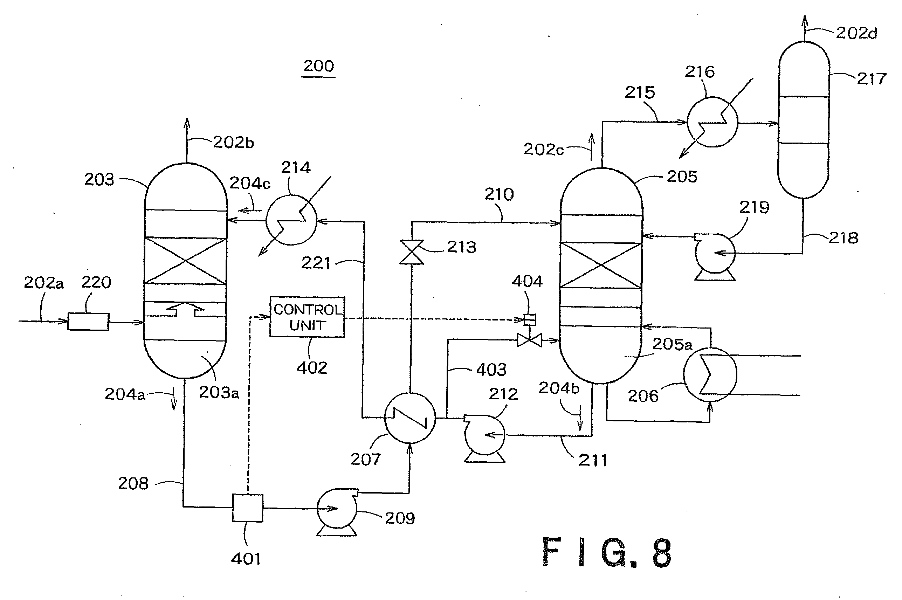

Fig. 8 shows the schematic structure of a carbon dioxide recovery system according to a third embodiment of the invention. This embodiment is different from the second embodiment shown in Fig. 5 in that a densimeter 401, a control unit 402, a lean liquid return line 403, and a regulating valve 404 are provided instead of the densimeter 301, the control unit 302, the rich liquid return line 303, and the regulating valve 304. Further, in this embodiment, a pump 212 is provided between a regeneration tower tank 205a and a branch point of the lean liquid return line 403 that is branched from a first lean liquid line 211. The same parts shown in Fig. 8 as those of the second embodiment shown in Fig. 5 are denoted by the same reference numerals, and the description thereof will be omitted.

Like the densimeter 301 of the second embodiment, the densimeter 401 is provided on a first rich liquid line 208 and measures the density of a rich liquid 204a in real time. As long as being capable of measuring the density of a liquid fluid in real time, any type of densimeter may be used as the densimeter 401. For example, a Coriolis mass flowmeter may be used. The densimeter 401 notifies the control unit 402 of the measured density of the rich liquid 204a.

The lean liquid return line 403 is connected to the first lean liquid line 211, and allows a lean liquid 204b to return to a regeneration tower 205. Here, the diameter of a pipe of the lean liquid return line 403 is set to about 1/2 to 1/5 of the diameter of a pipe of the first lean liquid line 211.

The regulating valve 404 is provided on the lean liquid return line 403, and can adjust the flow rate of the lean liquid 204b returning to a regeneration tower 205 by the opening thereof. The control unit 402 controls the opening of the regulating valve 404 on the basis of the density of the rich liquid 204a.

A method of controlling the opening of the regulating valve 404 by the control unit 402 according to this embodiment will be described.

If the density of the rich liquid 204a notified by the densimeter 401 is lower than a predetermined value, that is, if the carbon dioxide content of the rich liquid 204a is low, a desired amount of carbon dioxide is not absorbed in the absorbent solution. For this reason, a target recovery rate of carbon dioxide is not secured. In this case, the control unit 402 increases the opening of the regulating valve 404 to increase the flow rate of the lean liquid return line 403.

Since the amount of the lean liquid 204b supplied to the absorption tower 203 is reduced and the amount of the absorbent solution flowing in the absorption tower 203 is reduced, the carbon dioxide content of the absorbent solution is increased, a desired amount of carbon dioxide can be absorbed in the absorbent solution, and the density of the rich liquid is increased. Accordingly, it is possible to secure a target recovery rate of carbon dioxide.

On the other hand, if the density of the rich liquid 204a notified by the densimeter 401 is higher than a predetermined value, that is, if the carbon dioxide content of the rich liquid 204a is high, carbon dioxide more than a desired amount is absorbed in the absorbent solution. For this reason, carbon dioxide cannot be sufficiently separated from the absorbent solution in the regeneration tower 205. In this case, the control unit 402 reduces the opening of the regulating valve 404 to reduce the flow rate of the lean liquid return line 403.

Since the amount of the lean liquid 204b supplied to the absorption tower 203 is increased and the amount of the absorbent solution flowing in the absorption tower 203 is increased, the carbon dioxide content of the absorbent solution is reduced and the density of the rich liquid is reduced. Accordingly, it is possible to sufficiently separate carbon dioxide from the absorbent solution in the regeneration tower 205 without increasing the amount of heat supplied to a reboiler 206.

In this embodiment, the flow rate of the lean liquid 204b returning to the regeneration tower 205 is adjusted according to the density of the rich liquid 204a as described above. Accordingly, the amount of heat supplied to the reboiler 206 is reduced while a target recovery rate of carbon dioxide is secured. Since the density of the rich liquid 204a is obtained in real time by the densimeter 401 as described above, it is possible to quickly reflect density measurement results on the control of the flow rate of the lean liquid 204b returning to the regeneration tower 205 and to improve the operation stability of the carbon dioxide recovery system.

### (Fourth embodiment)

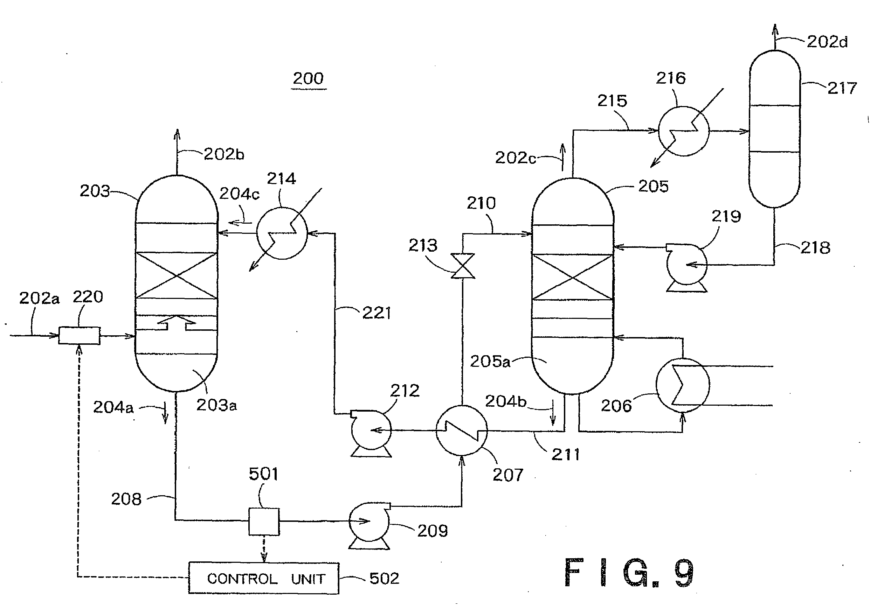

Fig. 9 shows the schematic structure of a carbon dioxide recovery system according to a fourth embodiment of the invention. This embodiment is different from the second embodiment shown in Fig. 5 in that a densimeter 501 and a control unit 502 are provided instead of the densimeter 301, the control unit 302, the rich liquid return line 303, and the regulating valve 304. The same parts shown in Fig. 9 as those of the second embodiment shown in Fig. 5 are denoted by the same reference numerals, and the description thereof will be omitted.

Like the densimeter 301 of the second embodiment, the densimeter 501 is provided on a first rich liquid line 208 and measures the density of a rich liquid 204a in real time. As long as being capable of measuring the density of a liquid fluid in real time, any type of densimeter may be used as the densimeter 501. For example, a Coriolis mass flowmeter may be used. The densimeter 501 notifies the control unit 502 of the measured density of the rich liquid 204a.

The control unit 502 controls the set temperature of a gas temperature controller 220 on the basis of the density of the rich liquid 204a.

If the density of the rich liquid 204a notified by the densimeter 501 is lower than a predetermined value, that is, if the carbon dioxide content of the rich liquid 204a is low, a desired amount of carbon dioxide is not absorbed in the absorbent solution. For this reason, a target recovery rate of carbon dioxide is not secured. In this case, the control unit 502 lowers the set temperature of the gas temperature controller 220.

When the temperature of a combustion exhaust gas 202a supplied to an absorption tower 203 is lowered, the carbon dioxide absorption rate of an absorbent solution is increased. Accordingly, a desired amount of carbon dioxide can be absorbed in the absorbent solution and the density of the rich liquid is increased. Accordingly, it is possible to secure a target recovery rate of carbon dioxide.

On the other hand, if the density of the rich liquid 204a notified by the densimeter 501 is higher than a predetermined value, that is, if the carbon dioxide content of the rich liquid 204a is high, carbon dioxide more than a desired amount is absorbed in the absorbent solution. For this reason, carbon dioxide cannot be sufficiently separated from the absorbent solution in a regeneration tower 205. In this case, the control unit 502 raises the set temperature of the gas temperature controller 220.

When the temperature of the combustion exhaust gas 202a supplied to the absorption tower 203 rises, the carbon dioxide absorption rate of the absorbent solution is reduced. Accordingly, the carbon dioxide content of the absorbent solution is reduced and the density of the rich liquid is reduced. Accordingly, it is possible to sufficiently separate carbon dioxide from the absorbent solution in the regeneration tower 205 without increasing the amount of heat supplied to a reboiler 206.

In this embodiment, the set temperature of the gas temperature controller 220 is adjusted according to the density of the rich liquid 204a as described above. Accordingly, the amount of heat supplied to the reboiler 206 is reduced while a target recovery rate of carbon dioxide is secured. Since the density of the rich liquid 204a is obtained in real time by the densimeter 501 as described above, it is possible to quickly reflect density measurement results on the control of the set temperature of the gas temperature controller 220 and to improve the operation stability of the carbon dioxide recovery system.

### (Fifth embodiment)

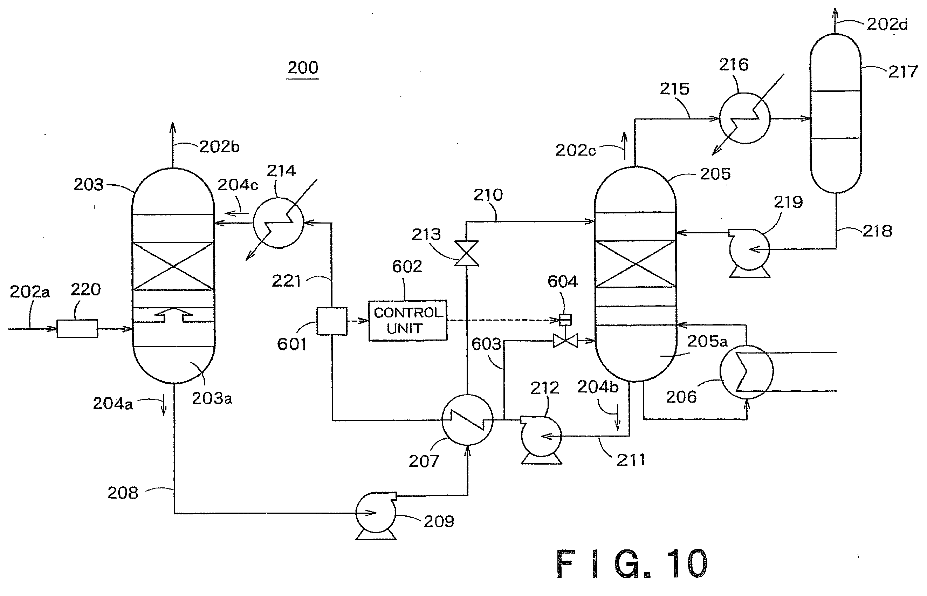

Fig. 10 shows the schematic structure of a carbon dioxide recovery system according to a fifth embodiment of the invention. This embodiment is different from the second embodiment shown in Fig. 5 in that a densimeter 601, a control unit 602, a lean liquid return line 603, and a regulating valve 604 are provided instead of the densimeter 301, the control unit 302, the rich liquid return line 303, and the regulating valve 304. Further, in this embodiment, a pump 212 is provided between a regeneration tower tank 205a and a branch point of the lean liquid return line 603 that is branched from a first lean liquid line 211. The same parts shown in Fig. 10 as those of the second embodiment shown in Fig. 5 are denoted by the same reference numerals, and the description thereof will be omitted.

The densimeter 601 is provided on a second lean liquid line 221, and measures the density of a lean liquid 204b in real time. As long as being capable of measuring the density of a liquid fluid in real time, any type of densimeter may be used as the densimeter 601. For example, a Coriolis mass flowmeter may be used. The densimeter 601 notifies the control unit 602 of the measured density of the lean liquid 204b.

The lean liquid return line 603 is connected to the first lean liquid line 211, and allows the lean liquid 204b to return to the lower portion of a regeneration tower 205. Here, the diameter of a pipe of the lean liquid return line 603 is set to about 1/2 to 1/5 of the diameter of a pipe of the first lean liquid line 211.

The regulating valve 604 is provided on the lean liquid return line 603, and can adjust the flow rate of the lean liquid 204b returning to the regeneration tower 205 by the opening thereof. The control unit 602 controls the opening of the regulating valve 604 on the basis of the density of the lean liquid 204b.

A method of controlling the opening of the regulating valve 604 by the control unit 602 according to this embodiment will be described.

If the density of the lean liquid 204b notified by the densimeter 601 is lower than a predetermined value, that is, if the carbon dioxide content of the lean liquid 204b is low, the separation of carbon dioxide caused by heating is excessively performed in the regeneration tower 205. In this case, the control unit 602 reduces the opening of the regulating valve 604 to reduce the flow rate of the lean liquid return line 603.

Accordingly, since the separation of carbon dioxide, which is caused by heating in the regeneration tower 205, is suppressed, the carbon dioxide content of the lean liquid 204b is increased and the density of the lean liquid is increased. Further, it is possible to set the carbon dioxide content of the lean liquid 204b, which is supplied to the absorption tower 203, to a desired amount. Meanwhile, in this case, the flow rate of the pump 212 may be reduced so that the flow rate of the lean liquid 204b supplied to the absorption tower 203 is not increased.

On the other hand, if the density of the lean liquid 204b notified by the densimeter 601 is higher than a predetermined value, that is, if the carbon dioxide content of the lean liquid 204b is high, carbon dioxide is not sufficiently separated from the absorbent solution in the regeneration tower 205. In this case, the control unit 602 increases the opening of the regulating valve 604 to increase the flow rate of the lean liquid return line 603.

Accordingly, since the separation of carbon dioxide, which is caused by heating in the regeneration tower 205, is facilitated, the carbon dioxide content of the lean liquid 204b is reduced and the density of the lean liquid is reduced. Further, it is possible to set the carbon dioxide content of the lean liquid 204b, which is supplied to the absorption tower 203, to a desired amount. Meanwhile, in this case, the flow rate of the pump 212 may be increased so that the flow rate of the lean liquid 204b supplied to the absorption tower 203 is not reduced.

In this embodiment, the flow rate of the lean liquid 204b returning to the regeneration tower 205 is adjusted according to the density of the lean liquid 204b as described above. Accordingly, the amount of heat supplied to the reboiler 206 is reduced while a target recovery rate of carbon dioxide is secured. Since the density of the lean liquid 204b is obtained in real time by the densimeter 601 as described above, it is possible to quickly reflect density measurement results on the control of the flow rate of the lean liquid 204b returning to the regeneration tower 205 and to improve the operation stability of the carbon dioxide recovery system.

### (Sixth embodiment)

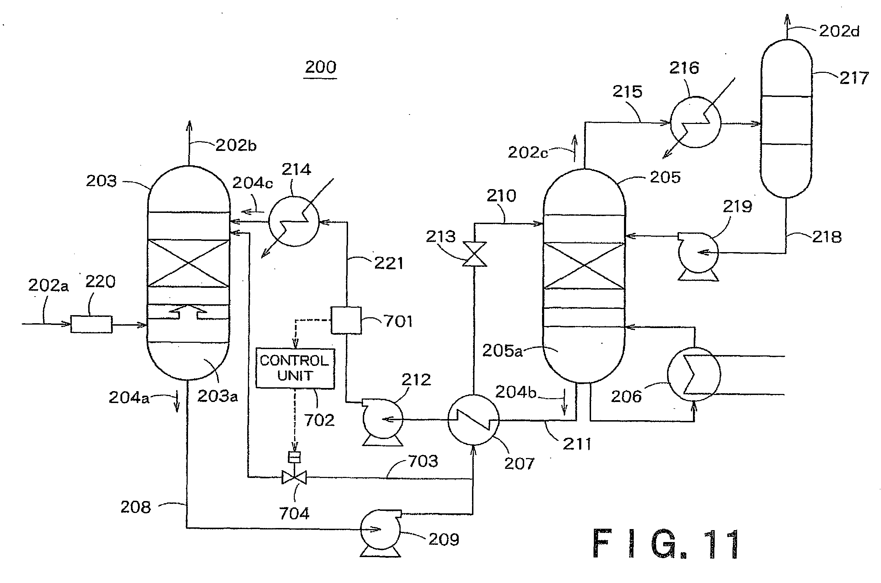

Fig. 11 shows the schematic structure of a carbon dioxide recovery system according to a sixth embodiment of the invention. This embodiment is different from the second embodiment shown in Fig. 5 in that a densimeter 701 and a control unit 702 are provided instead of the densimeter 301 and the control unit 302. The same parts shown in Fig. 11 as those of the second embodiment shown in Fig. 5 are denoted by the same reference numerals, and the description thereof will be omitted. Meanwhile, a rich liquid return line 703 and a regulating valve 704 have the same structure as the structure of the rich liquid return line 303 and the regulating valve 304 shown in Fig. 5.

The densimeter 701 is provided on a second lean liquid line 221, and measures the density of a lean liquid 204b in real time. As long as being capable of measuring the density of a liquid fluid in real time, any type of densimeter may be used as the densimeter 701. For example, a Coriolis mass flowmeter may be used. The densimeter 701 notifies the control unit 702 of the measured density of the lean liquid 204b. The control unit 702 controls the opening of a regulating valve 704 on the basis of the density of the lean liquid 204b.

A method of controlling the opening of the regulating valve 704 by the control unit 702 according to this embodiment will be described.

If the density of the lean liquid 204b notified by the densimeter 701 is lower than a predetermined value, that is, if the carbon dioxide content of the lean liquid 204b is low, carbon dioxide more than a desired amount is separated from an absorbent solution in a regeneration tower 205. In this case, the control unit 702 reduces the opening of the regulating valve 704 to reduce the flow rate of the rich liquid return line 703.

Accordingly, since the amount of a rich liquid 204a supplied to the regeneration tower 205 is increased and the amount of the absorbent solution flowing in the regeneration tower 205 is increased, the carbon dioxide content of the lean liquid 204b is increased and the density of the lean liquid is increased. Since the amount of the rich liquid 204a to be supplied corresponds to the amount of heat supplied to the reboiler 206, the carbon dioxide content of the lean liquid 204b is set to a desired amount and it is possible to secure a target recovery rate of carbon dioxide.

On the other hand, if the density of the lean liquid 204b notified by the densimeter 701 is higher than a predetermined value, that is, if the carbon dioxide content of the lean liquid 204b is high, carbon dioxide is not sufficiently separated from the absorbent solution in the regeneration tower 205. In this case, the control unit 702 increases the opening of the regulating valve 704 to increase the flow rate of the rich liquid return line 703.

Accordingly, since the amount of the rich liquid 204a supplied to the regeneration tower 205 is reduced and the amount of the absorbent solution flowing in the regeneration tower 205 is reduced, carbon dioxide is sufficiently separated from the absorbent solution in the regeneration tower 205 and the density of the lean liquid 204b is reduced. It is possible to sufficiently separate carbon dioxide from the absorbent solution in the regeneration tower 205 without increasing the amount of heat supplied to the reboiler 206.

In this embodiment, the flow rate of the rich liquid 204a returning to the absorption tower 203 is adjusted according to the density of the lean liquid 204b as described above. Accordingly, the amount of heat supplied to the reboiler 206 is reduced while a target recovery rate of carbon dioxide is secured. Since the density of the lean liquid 204b is obtained in real time by the densimeter 701 as described above, it is possible to quickly reflect density measurement results on the control of the flow rate of the rich liquid 204a returning to the absorption tower 203 and to improve the operation stability of the carbon dioxide recovery system.

### (Seventh embodiment)

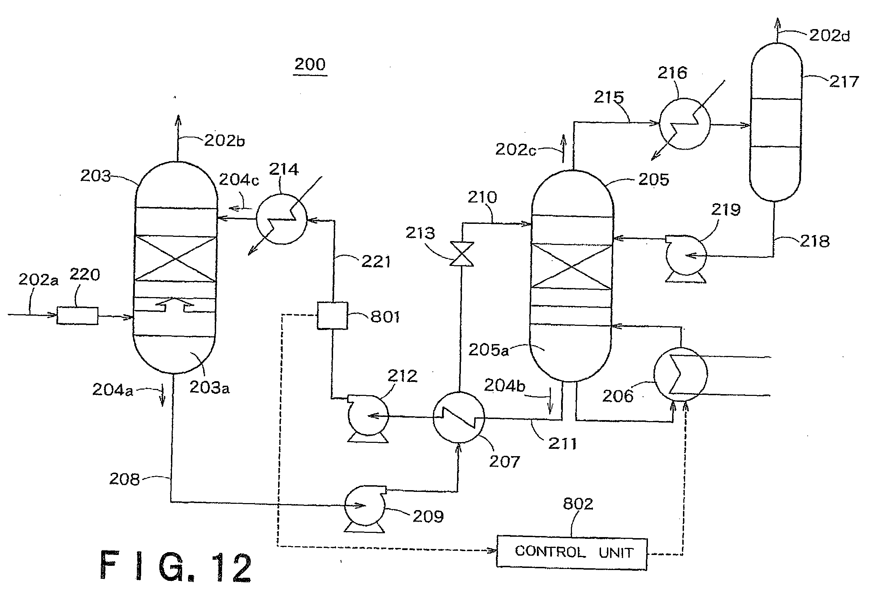

Fig. 12 shows the schematic structure of a carbon dioxide recovery system according to a seventh embodiment of the invention. This embodiment is different from the second embodiment shown in Fig. 5 in that a densimeter 801 and a control unit 802 are provided instead of the densimeter 301, the control unit 302, the rich liquid return line 303, and the regulating valve 304. The same parts shown in Fig. 12 as those of the second embodiment shown in Fig. 5 are denoted by the same reference numerals, and the description thereof will be omitted.

The densimeter 801 is provided on a second lean liquid line 221 and measures the density of a lean liquid 204b in real time. As long as being capable of measuring the density of a liquid fluid in real time, any type of densimeter may be used as the densimeter 801. For example, a Coriolis mass flowmeter may be used. The densimeter 801 notifies the control unit 802 of the measured density of the lean liquid 204b. The control unit 802 controls the set temperature of (the amount of heat supplied to) a reboiler 206 on the basis of the density of the lean liquid 204b.

A method of controlling the set temperature of the reboiler 206 by the control unit 802 according to this embodiment will be described.

If the density of the lean liquid 204b notified by the densimeter 801 is lower than a predetermined value, that is, if the carbon dioxide content of the lean liquid 204b is low, the control unit 802 lowers the set temperature of the reboiler 206.

Accordingly, the carbon dioxide content of the lean liquid 204b is increased and the density of the lean liquid is increased. The carbon dioxide content of the lean liquid 204b is set to a desired amount and it is possible to secure a target recovery rate of carbon dioxide. Further, it is possible to reduce the amount of heat supplied to the reboiler 206.

On the other hand, if the density of the lean liquid 204b notified by the densimeter 801 is higher than a predetermined value, that is, if the carbon dioxide content of the lean liquid 204b is high, carbon dioxide is not sufficiently separated from the absorbent solution in the regeneration tower 205. In this case, the control unit 802 raises the set temperature of the reboiler 206.

Accordingly, carbon dioxide is sufficiently separated from the absorbent solution in the regeneration tower 205 and the density of the lean liquid 204b is reduced. The carbon dioxide content of the lean liquid 204b is set to a desired amount and it is possible to secure a target recovery rate of carbon dioxide.

In this embodiment, the set temperature of the reboiler 206 is adjusted according to the density of the lean liquid 204b as described above. Accordingly, the amount of heat supplied to the reboiler 206 is reduced while a target recovery rate of carbon dioxide is secured. Since the density of the lean liquid 204b is obtained in real time by the densimeter 801 as described above, it is possible to quickly reflect density measurement results on the control of the set temperature of the reboiler 206 and to improve the operation stability of the carbon dioxide recovery system.

In the above-mentioned second to seventh embodiments, the density of the absorbent solution has been measured in real time and carbon dioxide content has been calculated (estimated) from this density. However, this method is premised on the fact that the composition of the absorbent solution is not changed. The reason for this is that a relationship between the carbon dioxide content and the density of the absorbent solution is also changed if the composition of the absorbent solution is changed.

Accordingly, it is preferable that the components of the absorbent solution be analyzed at an interval of a predetermined time (for example, 15 minutes) by the measurement device according to the first embodiment and a relationship between the carbon dioxide content and the density of the absorbent solution be corrected on the basis of the results of the analysis.

For example, the control units 302 to 802 acquire the detection results of the detection unit 7 of the measurement device, and calculate a relationship between the carbon dioxide content and the density of the absorbent solution. Further, the control units 302 to 802 calculate the range of the density of the absorbent solution that corresponds to the preferred carbon dioxide content shown in Fig. 7. The control units control the amount of the absorbent solution returning to the absorption tower 203 or the regeneration tower 205, the set temperature of the gas temperature controller 220, or the set temperature of the reboiler 206 if the measurement results of the densimeters 301 to 801 are out of this range.

Since the measurement device according to the first embodiment is used in combination as described above, it is possible to further accurately obtain the carbon dioxide content of the absorbent solution and to further improve the operation stability of the carbon dioxide recovery system.

Examples where an organic solution such as an amine compound aqueous solution is used as the absorbent solution have been described in the above-mentioned embodiments. However, even though an organic solvent, which does not include water, is used as the absorbent solution, moisture absorbed from the combustion exhaust gas is included in the absorbent solution circulating through the carbon dioxide recovery system. Accordingly, the absorbent solution of which components are to be analyzed by the measurement device may be regarded as an organic solution.

Meanwhile, the invention is not limited to the above-mentioned embodiments as it is, and may be embodied by the modifications of the elements within the range that does not depart from the scope of the invention when being embodied. Further, various inventions may be made by the appropriate combination of the plurality of elements disclosed in the above-mentioned embodiment. For example, some elements may be removed from all elements disclosed in the embodiment. Furthermore, the elements of the different embodiments may be appropriately combined.

### DESCRIPTION OF REFERENCE NUMERALS

- 1: AUTOMATIC FIXED AMOUNT COLLECTING UNIT
- 2: GASIFICATION UNIT
- 3: ORGANIC GAS RETENTION UNIT
- 4: FLOW PASSAGE SWITCHING UNIT
- 5: INORGANIC GAS SEPARATION UNIT
- 6: ORGANIC GAS SEPARATION UNIT
- 7: DETECTION UNIT
- 8: CONSTANT TEMPERATURE UNIT
- 301,401,501,601,701,801: DENSIMETER
- 302,402,502,602,702,802: CONTROL UNIT

## Claims

1. A measurement device comprising:
a gasification unit (2) that gasifies an organic solution in which an inorganic gas has been dissolved and discharges the gasified organic solution together with a carrier gas;
an organic gas retention unit (3) that is supplied with a gas discharged from the gasification unit (2), retains an organic gas and allows an inorganic gas to pass therethrough at a first temperature, and discharges the retained organic gas at a second temperature higher than the first temperature;
an inorganic gas separation unit (5) that separates inorganic components contained in the inorganic gas having passed through the organic gas retention unit (3), and discharges the inorganic components;
an organic gas separation unit (6) that separates organic components contained in the organic gas discharged from the organic gas retention unit (3), and discharges the organic components; and
a detection unit (7) that detects the inorganic components discharged from the inorganic gas separation unit (5) and the organic components discharged from the organic gas separation unit (6).

2. The measurement device according to claim 1, further comprising a flow passage switching unit (4) that supplies the inorganic gas having passed through the organic gas retention (3) unit to the inorganic gas separation unit (5), supplies a carrier gas to the organic gas separation unit (6), supplies the organic gas discharged from the organic gas retention unit (3) to the organic gas separation unit (6), and supplies a carrier gas to the inorganic gas separation unit (5).

3. The measurement device according to claim 2, further comprising a constant temperature unit (8) that receives the organic gas retention unit (3), the flow passage switching unit (4), the inorganic gas separation unit (5), and the organic gas separation unit (6), and maintains the organic gas retention unit (3), the flow passage switching unit, the inorganic gas separation unit (5), and the organic gas separation unit at a predetermined temperature.

4. The measurement device according to claim 2 or 3,
wherein the inorganic gas separation unit (5) discharges water vapor at a third temperature that is higher than the first temperature and lower than the second temperature, and the flow passage switching unit (4) switches a supply destination of the gas, which is discharged from the organic gas retention unit (3), to the organic gas separation unit (6) from the inorganic gas separation unit (5) as temperature rises from the first temperature to the third temperature.

5. The measurement device according to any one of claims 1 to 4,
wherein the organic solution is an absorbent solution circulating through a carbon dioxide recovery system including an absorption tower that allows carbon dioxide contained in a combustion exhaust gas to be absorbed in the absorbent solution and a regeneration tower (105) that is supplied with the absorbent solution having absorbed carbon dioxide from the absorption tower, discharges a carbon dioxide gas containing steam from the absorbent solution, and regenerates the absorbent solution, and
the detection unit (7) detects the carbon dioxide content of the absorbent solution.

6. A measurement method of measuring components of an organic solution in which an inorganic gas has been dissolved by a measurement device that includes a gasification unit, an organic gas retention unit (3), a flow passage switching unit (4), an inorganic gas separation unit (5), an organic gas separation unit (6), and a detection unit (7),
wherein the gasification unit (2) gasifies the organic solution and discharges the gasified organic solution together with a carrier gas,
the organic gas retention unit (3) retains an organic gas contained in the gas discharged from the gasification unit (2) and allows an inorganic gas to pass therethrough at a first temperature,
the inorganic gas separation unit (5) separates inorganic components contained in the inorganic gas, which has passed through the organic gas retention unit (3), and discharges the inorganic components, at a third temperature that is higher than the first temperature and lower than a second temperature where the organic gas retention unit (3) discharges the retained organic gas,
the detection unit (7) detects the inorganic components discharged from the inorganic gas separation unit (5),
the organic gas retention unit (3) discharges the organic gas at the second temperature,
the organic gas separation unit (6) separates organic components contained in the organic gas discharged from the organic gas retention unit (3), and discharges the organic components, and
the detection unit (7) detects the organic components discharged from the organic gas separation unit (6).

7. A carbon dioxide recovery system comprising:
an absorption tower (103) that allows carbon dioxide contained in a combustion exhaust gas to be absorbed in an absorbent solution and discharges the absorbent solution containing carbon dioxide;
a regeneration tower (105) that is supplied with the absorbent solution discharged from the absorption tower (103), removes a carbon dioxide gas containing steam from the absorbent solution, regenerates the absorbent solution, and discharges the absorbent solution;
a regenerative heat exchanger (107) that is provided between the absorption tower (103) and the regeneration tower (105) and heats the absorbent solution, which is discharged from the absorption tower and supplied to the regeneration tower, by using an absorbent solution, which is discharged from the regeneration tower and supplied to the absorption tower, as a heat source;
a densimeter (301) that measures the density of an absorbent solution discharged from the absorption tower or an absorbent solution discharged from the regeneration tower (105) ;
a measurement device according to claim 1, wherein the gasification unit (2) is configured to gasify a unit of the absorbent solution and to discharge the gasified absorbent solution together with the carrier gas;
and
a control unit (302) that controls the amount of an absorbent solution which is discharged from the absorption tower (103) and returns to the absorption tower or the amount of an absorbent solution which is discharged from the regeneration tower (105) and returns to the regeneration tower on the basis of the density measured by the densimeter and detection results of the detection unit (7).

8. The carbon dioxide recovery system according to claim 7, further comprising:
an absorbent solution return line through which the absorbent solution discharged from the absorption tower (103) returns to the absorption tower; and
a regulating valve that adjusts the flow rate of the absorbent solution return line,
wherein the densimeter (301) measures the density of the absorbent solution discharged from the absorption tower (103), and
the control unit (302) calculates first and second thresholds on the basis of the detection results of the detection unit (7), controls the regulating valve so as to increase the flow rate of the absorbent solution return line when the density is lower than the first threshold, and controls the regulating valve so as to reduce the flow rate of the absorbent solution return line when the density is higher than the second threshold.

9. The carbon dioxide recovery system according to claim 7, further comprising:
an absorbent solution return line through which the absorbent solution discharged from the regeneration tower returns to the regeneration tower (105); and
a regulating valve that adjusts the flow rate of the absorbent solution return line,
wherein the densimeter (301) measures the density of the absorbent solution discharged from the absorption tower (103), and
the control unit (302) calculates first and second thresholds on the basis of the detection results of the detection unit (7), controls the regulating valve so as to increase the flow rate of the absorbent solution return line when the density is lower than the first threshold, and controls the regulating valve so as to reduce the flow rate of the absorbent solution return line when the density is higher than the second threshold.

10. The carbon dioxide recovery system according to claim 7, further comprising:
an absorbent solution return line through which the absorbent solution discharged from the regeneration tower (105) returns to the regeneration tower; and
a regulating valve that adjusts the flow rate of the absorbent solution return line,
wherein the densimeter (301) measures the density of the absorbent solution discharged from the regeneration tower, and the control unit (302) calculates first and second thresholds on the basis of the detection results of the detection unit (7), controls the regulating valve so as to reduce the flow rate of the absorbent solution return line when the density is lower than the first threshold, and controls the regulating valve so as to increase the flow rate of the absorbent solution return line when the density is higher than the second threshold.

11. The carbon dioxide recovery system according to claim 7, further comprising:
an absorbent solution return line through which the absorbent solution discharged from the absorption tower (103) returns to the absorption tower; and
a regulating valve that adjusts the flow rate of the absorbent solution return line,
wherein the densimeter (301) measures the density of the absorbent solution discharged from the regeneration tower (105), and
the control unit (302) calculates first and second thresholds on the basis of the detection results of the detection unit (7), controls the regulating valve so as to reduce the flow rate of the absorbent solution return line when the density is lower than the first threshold, and controls the regulating valve so as to increase the flow rate of the absorbent solution return line when the density is higher than the second threshold.

12. The carbon dioxide recovery system according to claim 7, further comprising:
a gas temperature controller that adjusts the temperature of a combustion exhaust gas and discharges the combustion exhaust gas, wherein
the absorption tower (103) allows carbon dioxide contained in the combustion exhaust gas discharged from the gas temperature controller to be absorbed in an absorbent solution and discharges the absorbent solution containing carbon dioxide,
the densimeter (301) measures the density of an absorbent solution discharged from the absorption tower, and
the control unit (302) calculates first and second thresholds on the basis of the detection results of the detection unit (7), performs a control so as to lower the set temperature of the gas temperature controller when the density is lower than the first threshold, and performs a control so as to raise the set temperature of the gas temperature controller when the density is higher than the second threshold.

13. The carbon dioxide recovery system according to claim 7, further comprising:
a reboiler that heats a unit of an absorbent solution stored in the regeneration tower (105), wherein
the densimeter (301) measures the density of an absorbent solution discharged from the regeneration tower, and
the control unit (302) calculates first and second thresholds on the basis of the detection results of the detection unit (7), performs a control so as to lower the set temperature of the reboiler when the density is lower than the first threshold, and performs a control so as to raise the set temperature of the reboiler when the density is higher than the second threshold.

14. The carbon dioxide recovery system according to any one of claims 7 to 13,
wherein the densimeter (301) includes a Coriolis mass flowmeter.

## Patentansprüche

1. Messeinrichtung mit:
einer Vergasungseinheit (2), die eine organische Lösung, in der ein anorganisches Gas gelöst wurde, vergast und die vergaste organische Lösung zusammen mit einem Trägergas abgibt;
einer Speichereinheit für organisches Gas (3), der ein Gas, das von der Vergasungseinheit (2) abgegeben wurde, zugeführt wird, und die ein organisches Gas speichert und es einem anorganischen Gas erlaubt, bei einer ersten Temperatur hindurch zu treten, und das gespeicherte organische Gas bei einer zweiten Temperatur, die höher als die erste Temperatur ist, abgibt;
einer Trenneinheit für anorganisches Gas (5), die anorganische Komponenten, die in dem anorganischen Gas, das die Speichereinheit für organisches Gas (3) durchtreten hat, enthalten sind, abtrennt, und die anorganischen Komponenten abgibt;
einer Trenneinheit für organisches Gas (6), die organischen Komponenten, die in dem organischen Gas, das von der Speichereinheit für organisches Gas (3) abgegeben wurde, enthalten sind, abtrennt, und die organischen Komponenten abgibt; und
einer Detektionseinheit (7), welche die anorganischen Komponenten, die von der Trenneinheit für anorganisches Gas (5) abgegeben wurden und die organischen Komponenten, die von der Trenneinheit für organisches Gas (6) abgegeben wurde, detektiert.

2. Messeinrichtung gemäß Anspruch 1, ferner mit einer Strömungskanalschalteinheit (4), die, der Trenneinheit für anorganisches Gas (5) das anorganische Gas, das die Speichereinheit für organisches Gas (3) durchtreten hat, zuführt, der Trenneinheit für organisches Gas (6) ein Trägergas zuführt, der Trenneinheit für organisches Gas (6) organisches Gas, das von der Speichereinheit für organisches Gas (3) abgegeben wurde, zuführt, und der Trenneinheit für anorganisches Gas (5) ein Trägergas zuführt.

3. Messeinrichtung nach Anspruch 2, ferner mit
einer Konstanttemperatureinheit (8), welche die Speichereinheit für organisches Gas (3), die Strömungskanalschalteinheit (4), die Trenneinheit für anorganisches Gas (5) und die Trenneinheit für organisches Gas (6) aufnimmt, und die Speichereinheit für organisches Gas (3), das Strömungskanalschaltelement, die Trenneinheit für anorganisches Gas (5) und die Trenneinheit für organisches Gas auf einer vorbestimmten Temperatur hält.

4. Messeinrichtung gemäß Anspruch 2 oder 3, wobei
die Trenneinheit für anorganisches Gas (5) Wasserdampf bei einer dritten Temperatur, die höher als die erste Temperatur und niedriger als die zweite Temperatur ist, abgibt, und die Strömungskanalschalteinheit (4) einen Zuführbestimmungsort des Gases, das von der Speichereinheit für organisches Gas (3) abgegeben wird, von der Trenneinheit für anorganisches Gas (5) zu der Trenneinheit für organisches Gas (6) umschaltet, wenn die Temperatur von der ersten Temperatur auf die dritte Temperatur ansteigt.

5. Messeinrichtung nach einem der Ansprüche 1 bis 4, wobei
die organische Lösung eine Absorptionslösung ist, die durch ein Kohlenstoffdioxidrückgewinnungssystem zirkuliert, das einen Absorptionsturm, der es Kohlenstoffdioxid, das in einem Verbrennungsabgas enthaltenen ist, erlaubt, in der Absorptionslösung absorbiert zu werden, und einen Rückgewinnungsturm (105) aufweist, dem die Absorptionslösung, die das Kohlenstoffdioxid aus dem Absorptionsturm absorbiert hat, zugeführt wird, und der ein Kohlenstoffdioxidgas, das Dampf enthält, aus der Absorptionslösung entfernt, und die Absorptionslösung zurückgewinnt, und wobei die Detektionseinheit (7) den Kohlenstoffdioxidgehalt der Absorptionslösung detektiert.

6. Messverfahren zum Messen von Komponenten einer organischen Lösung, in der ein anorganisches Gas gelöst wurde, durch eine Messeinrichtung, die eine Vergasungseinheit, eine Speichereinheit für organisches Gas (3), eine Strömungswegschalteinheit (4), eine Trenneinheit für anorganisches Gas (5), eine Trenneinheit für organisches Gas (6) und eine Detektionseinheit (7) aufweist, wobei
die Vergasungseinheit (2) die organische Lösung vergast und die vergaste organische Lösung zusammen mit einem Trägergas abgibt,
die Speichereinheit für organisches Gas (3) ein organisches Gas, das in dem Gas, das von der Vergasungseinheit (2) abgegeben wurde, enthalten ist, zurückhält und es einem anorganischen Gas erlaubt, bei einer ersten Temperatur dort hindurch zu treten,
die Trenneinheit für anorganisches Gas (5) die anorganischen Komponenten, die in dem anorganischen Gas, das die Speichereinheit für organisches Gas (3) durchtreten hat, abtrennt, und die anorganischen Komponenten bei einer dritten Temperatur abgibt, die höher als die erste Temperatur und niedriger als eine zweite Temperatur ist, bei der die Speichereinheit für organisches Gas (3) das gespeicherte organische Gas abgibt,
die Detektionseinheit (7) die anorganischen Komponenten, die von der Trenneinheit für anorganisches Gas (5) abgegeben wurde, detektiert,
die Speichereinheit für organisches Gas (3) das organische Gas bei der zweiten Temperatur abgibt,
die Trenneinheit für organisches Gas (6) die organischen Komponenten, die in dem organischen Gas, das von der Speichereinheit für organisches Gas (3) abgegeben wurde, enthalten sind, abtrennt, und die organischen Komponenten abgibt, und
die Detektionseinheit (7) die organischen Komponenten, die von der Trenneinheit für organisches Gas abgegeben wurde, detektiert.

7. Kohlenstoffdioxidrückgewinnungssystem, mit:
einem Absorptionsturm (103), der es Kohlenstoffdioxid, das in einem Verbrennungsabgas enthalten ist, erlaubt, in einer Absorptionslösung absorbiert zu werden und die Absorptionslösung, die Kohlenstoffdioxid enthält, abgibt;
einem Rückgewinnungsturm (105), dem die Absorptionslösung, die von dem Absorptionsturm (103) abgegeben wurde, zugeführt wird, und der ein Kohlenstoffdioxidgas, das Dampf enthält, aus der Absorptionslösung entfernt, die Absorptionslösung rückgewinnt, und die Absorptionslösung abgibt;
einem Rückgewinnungswärmetauscher (107), der zwischen dem Absorptionsturm (103) und der Rückgewinnungsturm (105) vorgesehen ist und die Absorptionslösung, die von dem Absorptionsturm abgegeben und dem Rückgewinnungsturm zugeführt wird, mittels einer Absorptionslösung, die von dem Rückgewinnungsturm abgegeben wird und dem Absorptionsturm zugeführt wird, als eine Wärmequelle, heizt;
einem Dichtemesser (301), der die Dichte einer Absorptionslösung, die von dem Absorptionsturm abgegeben wurde oder einer Absorptionslösung, die von dem Rückgewinnungsturm (105) abgegeben wurde, misst;
einer Messeinrichtung nach Anspruch 1, wobei die Vergasungseinheit (2) ausgestaltet ist, um einen Teil der Absorptionslösung zu vergasen und, um die vergaste Absorptionslösung zusammen mit dem Trägergas abzugeben; und
einer Steuereinheit (302), welche die Menge einer Absorptionslösung, die von dem Absorptionsturm (103) abgegeben wird und zu dem Absorptionsturm zurückkehrt oder die Menge einer Absorptionslösung, die von dem Rückgewinnungsturm (105) abgegeben wird und zu dem Rückgewinnungsturm zurückkehrt, auf Grundlage der Dichte, die durch den Dichtemesser gemessen wurde und Detektionsergebnissen der Detektionseinheit (7), steuert.

8. Kohlenstoffdioxidrückgewinnungssystem nach Anspruch 7, ferner mit:
einer Absorptionslösungsrückführleitung durch welche die Absorptionslösung, die von dem Absorptionsturm (103) abgegeben wurde, zu dem Absorptionsturm zurückkehrt; und
einem Regelventil, das den Durchfluss der Absorptionslösungsrückführleitung einstellt,
wobei der Dichtemesser (301) die Dichte der Absorptionslösung, die von dem Absorptionsturm (103) abgegeben wurde, misst, und
die Steuereinheit (302) erste und zweite Schwellenwerte auf Grundlage der Detektionsergebnisse der Detektionseinheit (7) berechnet, das Regelventil derart steuert, dass der Durchfluss der Absorptionslösungsrückführleitung erhöht wird, wenn die Dichte niedriger als der erste Schwellenwert ist, und das Regelventil derart steuert, dass der Durchfluss der Absorptionslösungsrückführleitung reduziert wird, wenn die Dichte höher als der zweite Schwellenwert ist.

9. Kohlenstoffdioxidrückgewinnungssystem nach Anspruch 7, ferner mit:
einer Absorptionslösungsrückführleitung, durch welche die Absorptionslösung, die von dem Rückgewinnungsturm abgegeben wurde, zu dem Rückgewinnungsturm (105) zurückkehrt; und
einem Regelventil, das den Durchfluss der Absorptionslösungsrückführleitung einstellt, wobei
der Dichtemesser (301) die Dichte der Absorptionslösung, die von dem Absorptionsturm (103) abgegeben wurde, misst, und
die Steuereinheit (302) erste und zweite Schwellenwerte auf Grundlage der Detektionsergebnisse der Detektionseinheit (7) berechnet, das Regelventil derart steuert, dass der Durchfluss der Absorptionslösungsrückführleitung erhöht wird, wenn die Dichte niedriger als der erste Schwellenwert ist, und das Regelventil derart steuert, dass der Durchfluss der Absorptionslösungsrückführleitung reduziert wird, wenn die Dichte höher als der zweite Schwellenwert ist.

10. Kohlenstoffdioxidrückgewinnungssystem nach Anspruch 7, ferner mit:
einer Absorptionslösungsrückführleitung, durch welche die Absorptionslösung, die von dem Rückgewinnungsturm (105) abgegeben wurde, zu dem Rückgewinnungsturm zurückkehrt; und
einem Regelventil, das den Durchfluss der Absorptionslösungsrückführleitung einstellt, wobei
der Dichtemesser (301) die Dichte der Absorptionslösung, die von dem Rückgewinnungsturm abgegeben wurde, misst, und
die Steuereinheit (302) erste und zweite Schwellenwerte auf Grundlage der Detektionsergebnisse der Detektionseinheit (7) berechnet, das Regelventil derart steuert, dass der Durchfluss der Absorptionslösungsrückführleitung reduziert wird, wenn die Dichte niedriger als der erste Schwellenwert ist, und das Regelventil derart steuert, dass der Durchfluss der Absorptionslösungsrückführleitung erhöht wird, wenn die Dichte höher als der zweite Schwellenwert ist.

11. Kohlenstoffdioxidrückgewinnungssystem nach Anspruch 7, ferner mit:
einer Absorptionslösungsrückführleitung, durch welche die Absorptionslösung, die von dem Absorptionsturm (103) abgegeben wurde, zu dem Absorptionsturm zurückkehrt; und
einem Regelventil, das den Durchfluss der Absorptionslösungsrückführleitung einstellt, wobei
der Dichtemesser (301) die Dichte der Absorptionslösung, die von dem Rückgewinnungsturm (105) abgegeben wurde, misst, und
die Steuereinheit (302) erste und zweite Schwellenwerte auf Grundlage der Detektionsergebnisse der Detektionseinheit (7) berechnet, das Regelventil derart steuert, dass der Durchfluss der Absorptionslösungsrückführleitung reduziert wird, wenn die Dichte niedriger als der erste Schwellenwert ist, und das Regelventil derart steuert, dass der Durchfluss der Absorptionslösungsrückführleitung erhöht wird, wenn die Dichte höher als der zweite Schwellenwert ist.

12. Kohlenstoffdioxidrückgewinnungssystem nach Anspruch 7, ferner mit:
einer Gastemperatursteuerung, welche die Temperatur eines Verbrennungsabgases einstellt und das Verbrennungsabgas abgibt, wobei
der Absorptionsturm (103), es Kohlenstoffdioxid, das in einem Verbrennungsabgas, das von dem Gastemperaturcontroller abgegeben wurde, enthalten ist, erlaubt, in einer Absorptionslösung absorbiert zu werden und die Absorptionslösung, die Kohlenstoffdioxid enthält, abgibt, wobei
der Dichtemesser (301) die Dichte einer Absorptionslösung, die von dem Absorptionsturm abgegeben wurde, misst, und
die Steuereinheit (302) erste und zweite Schwellenwerte auf Grundlage der Detektionsergebnisse der Detektionseinheit (7) berechnet, eine Steuerung derart durchführt, dass die Solltemperatur der Gastemperatursteuerung verringert wird, wenn die Dichte niedriger als der erste Schwellenwert ist, und eine Steuerung derart durchführt, dass die Solltemperatur der Gastemperatursteuerung erhöht wird, wenn die Dichte höher als der zweite Schwellenwert ist.

13. Kohlenstoffdioxidrückgewinnungssystem nach Anspruch 7, ferner mit:
einem Verdampfer, der einen Teil der Absorptionslösung, die in dem Rückgewinnungsturm (105) gespeichert ist, erwärmt, wobei
der Dichtemesser (301) die Dichte einer Absorptionslösung, die von dem Rückgewinnungsturm abgegeben wurde, misst, und
die Steuereinheit (302) erste und zweite Schwellenwerte auf Grundlage der Detektionsergebnisse der Detektionseinheit (7) berechnet, eine Steuerung derart durchführt, dass die Solltemperatur des Verdampfers verringert wird, wenn die Dichte niedriger als der erste Schwellenwert ist, und eine Steuerung derart durchführt, dass die Solltemperatur des Verdampfers erhöht wird, wenn die Dichte höher als der zweite Schwellenwert ist.

14. Kohlenstoffdioxidrückgewinnungssystem nach einem der Ansprüche 7 bis 13, wobei
der Dichtemesser (301) einen Coriolis-Massendurchflussmesser aufweist.

## Revendications

1. Dispositif de mesure comprenant :
une unité de gazéification (2) qui gazéifie une solution organique dans laquelle un gaz inorganique a été dissous et évacue la solution organique gazéifiée conjointement à un gaz porteur ;
une unité de rétention de gaz organique (3) qui est alimentée en gaz évacué de l'unité de gazéification (2), retient un gaz organique et permet à un gaz inorganique de passer à travers elle à une première température, et évacue le gaz organique retenu à une deuxième température supérieure à la première température ;
une unité de séparation de gaz inorganique (5) qui sépare des composants inorganiques contenus dans le gaz inorganique qui est passé à travers l'unité de rétention de gaz organique (3), et évacue les composants inorganiques ;
une unité de séparation de gaz organique (6) qui sépare des composants organiques contenus dans le gaz organique évacué de l'unité de rétention de gaz organique (3), et évacue les composants organiques ; et
une unité de détection (7) qui détecte les composants inorganiques évacués de l'unité de séparation de gaz inorganique (5) et les composants organiques évacués de l'unité de séparation de gaz organique (6).

2. Dispositif de mesure selon la revendication 1, comprenant en outre une unité de commutation de passage d'écoulement (4) qui alimente un gaz inorganique qui est passé à travers l'unité de rétention de gaz organique (3) vers l'unité de séparation de gaz inorganique (5), alimente un gaz porteur vers l'unité de séparation de gaz organique (6), alimente le gaz organique évacué l'unité de rétention de gaz organique (3) vers l'unité de séparation de gaz organique (6), et alimente un gaz porteur vers l'unité de séparation de gaz inorganique (5).

3. Dispositif de mesure selon la revendication 2, comprenant en outre une unité de température constante (8) qui reçoit l'unité de rétention de gaz organique (3), l'unité de commutation de passage d'écoulement (4), l'unité de séparation de gaz inorganique (5) et l'unité de séparation de gaz organique (6), et maintient l'unité de rétention de gaz organique (3), l'unité de commutation de passage d'écoulement, l'unité de séparation de gaz inorganique (5) et l'unité de séparation de gaz organique à une température prédéterminée.

4. Dispositif de mesure selon la revendication 2 ou 3, dans lequel l'unité de séparation de gaz inorganique (5) évacue de la vapeur d'eau à une troisième température qui est supérieure à la première température et inférieure à la deuxième température, et l'unité de commutation de passage d'écoulement (4) commute une destination d'alimentation du gaz, qui est évacué de l'unité de rétention de gaz organique (3), vers l'unité de séparation de gaz organique (6) depuis l'unité de séparation de gaz inorganique (5) lorsqu'une température s'élève de la première température à la troisième température.

5. Dispositif de mesure selon l'une quelconque des revendications 1 à 4,
dans lequel la solution organique est une solution absorbante circulant à travers un système de récupération de dioxyde de carbone comprenant une tour d'absorption qui permet au dioxyde de carbone contenu dans un gaz d'échappement de combustion d'être absorbé dans la solution absorbante et une tour de régénération (105) qui est alimentée en solution absorbante ayant absorbé du dioxyde de carbone de la tour d'absorption, évacue un gaz dioxyde de carbone contenant de la vapeur de la solution absorbante, et régénère la solution absorbante, et
l'unité de détection (7) détecte la teneur en dioxyde de carbone de la solution absorbante.

6. Procédé de mesure de composants de mesure d'une solution organique dans laquelle un gaz organique a été dissous par un dispositif de mesure qui comprend une unité de gazéification, une unité de rétention de gaz organique (3), une unité de commutation de passage d'écoulement (4), une unité de séparation de gaz inorganique (5), une unité de séparation de gaz organique (6) et une unité de détection (7),
dans lequel l'unité de gazéification (2) gazéifie la solution organique et évacue la solution organique gazéifiée conjointement à un gaz porteur,
l'unité de rétention de gaz organique (3) retient un gaz organique contenu dans le gaz évacué de l'unité de gazéification (2) et permet à un gaz inorganique de passer à travers elle à une première température,
l'unité de séparation de gaz inorganique (5) sépare des composants inorganiques contenus dans le gaz inorganique, qui est passé à travers l'unité de rétention de gaz organique (3), et évacue les composants inorganiques, à une troisième température qui est supérieure à la première température et inférieure à une deuxième température où l'unité de rétention de gaz organique (3) évacue le gaz organique retenu,
l'unité de détection (7) détecte les composants inorganiques évacués de l'unité de séparation de gaz inorganique (5),
l'unité de rétention de gaz organique (3) évacue le gaz organique à la deuxième température,
l'unité de séparation de gaz organique (6) sépare des composants organiques contenus dans le gaz organique évacué de l'unité de rétention de gaz organique (3), et évacue les composants organiques, et
l'unité de détection (7) détecte les composants organiques évacués de l'unité de séparation de gaz organique (6).

7. Système de récupération de dioxyde de carbone comprenant :
une tour d'absorption (103) qui permet au dioxyde de carbone contenu dans un gaz d'évacuation de combustion d'être absorbé dans une solution absorbante et évacue la solution absorbante contenant le dioxyde de carbone ;
une tour de régénération (105) qui est alimentée en solution absorbante évacuée de la tour d'absorption (103), élimine un gaz dioxyde de carbone contenant de la vapeur de la solution absorbante, régénère la solution absorbante, et évacue la solution absorbante ;
un échangeur de chaleur régénératif (107), qui est agencé entre la tour d'absorption (103) et la tour de régénération (105) et chauffe la solution absorbante qui est évacuée de la tour d'absorption et amenée à la tour de régénération, en utilisant une solution absorbante qui est évacuée de la tour de régénération et amenée à la tour d'absorption, en tant que source de chaleur ;
un densimètre (301) qui mesure la densité d'une solution absorbante évacuée de la tour d'absorption ou d'une solution absorbante évacuée de la tour de régénération (105) ;
un dispositif de mesure selon la revendication 1, dans lequel l'unité de gazéification (2) est configurée pour gazéifier une unité de la solution absorbante et pour évacuer la solution absorbante gazéifiée conjointement au gaz porteur ;
et
une unité de commande (302) qui commande la quantité d'une solution absorbante qui est évacuée de la tour d'absorption (103) et retourne à la tour d'absorption ou la quantité d'une solution absorbante qui est évacuée de la tour de régénération (105) et retourne à la tour de régénération sur la base de la densité mesurée par le densimètre et des résultats de détection de l'unité de détection (7).

8. Système de récupération de dioxyde de carbone selon la revendication 7, comprenant en outre :
une conduite de retour de solution absorbante à travers laquelle la solution absorbante évacuée de la tour d'absorption (103) retourne à la tour d'absorption ; et
une soupape de régulation qui ajuste le débit de la conduite de retour de solution absorbante,
dans lequel le densimètre (301) mesure la densité de la solution absorbante évacuée de la tour d'absorption (103), et
l'unité de commande (302) calcule des premier et second seuils sur la base des résultats de détection de l'unité de détection (7), commande la soupape de régulation de façon à augmenter le débit de la conduite de retour de solution absorbante lorsque la densité est inférieure au premier seuil, et commande la soupape de régulation de façon à réduire le débit de la conduite de retour de solution absorbante lorsque la densité est supérieure au second seuil.

9. Système de récupération de dioxyde de carbone selon la revendication 7, comprenant en outre :
une conduite de retour de solution absorbante à travers laquelle la solution absorbante évacuée de la tour de régénération retourne à la tour de régénération (105) ; et
une soupape de régulation qui ajuste le débit de la conduite de retour de solution absorbante,
dans lequel le densimètre (301) mesure la densité de la solution absorbante évacuée de la tour d'absorption (103), et
l'unité de commande (302) calcule des premier et second seuils en se basant sur les résultats de détection de l'unité de détection (7), commande la soupape de régulation de façon à augmenter le débit de la conduite de retour de solution absorbante lorsque la densité est inférieure au premier seuil, et commande la soupape de régulation de façon à réduire le débit de la conduite de retour de solution absorbante lorsque la densité est supérieure au second seuil.

10. Système de récupération de dioxyde de carbone selon la revendication 7, comprenant en outre :
une conduite de retour de solution absorbante à travers laquelle la solution absorbante évacuée de la tour de régénération (105) retourne à la tour de régénération ; et
une soupape de régulation qui ajuste le débit de la conduite de retour de solution absorbante,
dans lequel le densimètre (301) mesure la densité de la solution absorbante évacuée de la tour de régénération, et l'unité de commande (302) calcule des premier et second seuils sur la base des résultats de détection de l'unité de détection (7), commande la soupape de régulation de façon à réduire le débit de la conduite de retour de solution absorbante lorsque la densité est inférieure au premier seuil, et commande la soupape de régulation de façon à augmenter le débit de la conduite de retour de solution absorbante lorsque la densité est supérieure au second seuil.

11. Système de récupération de dioxyde de carbone selon la revendication 7, comprenant en outre :
une conduite de retour de solution absorbante à travers laquelle la solution absorbante évacuée de la tour d'absorption (103) retourne à la tour d'absorption ; et
une soupape de régulation qui ajuste le débit de la conduite de retour de solution absorbante,
dans lequel le densimètre (301) mesure la densité de la solution absorbante évacuée de la tour de régénération (105), et
l'unité de commande (302) calcule des premier et second seuils sur la base des résultats de détection de l'unité de détection (7), commande la soupape de régulation de façon à réduire le débit de la conduite de retour de solution absorbante lorsque la densité est inférieure au premier seuil, et commande la soupape de régulation de façon à augmenter le débit de la conduite de retour de solution absorbante lorsque la densité est supérieure au second seuil.

12. Système de récupération de dioxyde de carbone selon la revendication 7, comprenant en outre :
un élément de commande de température de gaz qui ajuste la température d'un gaz d'échappement de combustion et évacue le gaz d'échappement de combustion, dans lequel
la tour d'absorption (103) permet au dioxyde de carbone contenu dans le gaz d'échappement de combustion évacué de l'élément de commande de température de gaz d'être absorbé dans une solution absorbante et évacue la solution absorbante contenant le dioxyde de carbone,
le densimètre (301) mesure la densité d'une solution absorbante évacuée de la tour d'absorption, et
l'unité de commande (302) calcule des premier et second seuils sur la base des résultats de détection de l'unité de détection (7), réalise une commande de façon à abaisser la température de consigne de l'élément de commande de température de gaz lorsque la densité est inférieure au premier seuil, et réalise une commande de façon à augmenter la température de consigne de l'élément de commande de température de gaz lorsque la densité est supérieure au second seuil.

13. Système de récupération de dioxyde de carbone selon la revendication 7, comprenant en outre :
un rebouilleur qui chauffe une unité d'une solution absorbante stockée dans la tour de régénération (105), dans lequel
le densimètre (301) mesure la densité d'une solution absorbante évacuée de la tour de régénération, et
l'unité de commande (302) calcule des premier et second seuils sur la base des résultats de détection de l'unité de détection (7), réalise une commande de façon à abaisser la température de consigne du rebouilleur lorsque la densité est inférieure au premier seuil, et réalise une commande de façon à augmenter la température de consigne du rebouilleur lorsque la densité est supérieure au second seuil.

14. Système de récupération de dioxyde de carbone selon l'une quelconque des revendications 7 à 13,
dans lequel le densimètre (301) comprend un débitmètre massique de Coriolis.
